# EUROPEAN PATENT APPLICATION

(11) **EP 4 530 358 A2**
(43) Date of publication of application: **02.04.2025**
(21) Application number: 25157119.6
(22) Date of filing: 15.09.2021
(51) Int. Cl.: C12Q 1/6837

(54) **METHODS OF RELEASING AN EXTENDED CAPTURE PROBE FROM A SUBSTRATE AND USES OF THE SAME**

(30) Priority: 15.09.2020 US 202063078754 P
(62) Divisional of application: 21789953.3
(71) Applicant: 10x Genomics, Inc., Pleasanton, CA 94588-3260 (US)
(72) Inventor: BENT, Zachary, Pleasanton, 94588-3260 (US); CHEW, Jennifer, Pleasanton, 94588-3260 (US); YIN, Yifeng, Pleasanton, 94588-3260 (US)
(74) Representative: J A Kemp LLP

(57) **Abstract**

Provided herein are methods of releasing an extended capture probe from a substrate and uses of the same.

## Description

### CROSS-REFERENCE TO RELATED APPLICATIONS

This application claims priority to U.S. Provisional Patent Application No. 63/078,754, filed on September 15, 2020, the entire contents of which are hereby incorporated by reference.

### BACKGROUND

Cells within a tissue of a subject have differences in cell morphology and/or function due to varied analyte levels (e.g., gene and/or protein expression) within the different cells. The specific position of a cell within a tissue (e.g., the cell's position relative to neighboring cells or the cell's position relative to the tissue microenvironment) can affect, e.g., the cell's morphology, differentiation, fate, viability, proliferation, behavior, and signaling and crosstalk with other cells in the tissue.

Spatial heterogeneity has been previously studied using techniques that only provide data for a small handful of analytes in the context of an intact tissue or a portion of a tissue, or provides substantial analyte data for dissociated tissue (i.e., single cells), but fail to provide information regarding the position of the single cell in a parent biological sample (e.g., tissue sample).

Currently, after the preparation of a biological sample on a spatially-barcoded array, the captured analytes are spatially-barcoded by reverse transcription to produce an extended capture probe (also called a first-strand cDNA). In order to prepare a library, the captured analyte is denatured from the extended capture probe and a double-stranded template switch oligonucleotide (TSO) is concatenated onto the 3' end of the extended capture probe. A polymerase enzyme can then be added which will generate a second strand from the extended capture probe that includes the spatial barcode information. This second strand can then be released from the extended capture probe of the spatially-barcoded array and transferred to a new tube for amplification and sequencing. Although this method works, it requires additional enzyme, primer, and buffer in order to synthesize the second strand from the extended capture probe.

### SUMMARY

Provided herein are methods for releasing an extended capture probe from a spatially-barcoded array. These methods provide for a reduction in both the time and costs associated with the generation and release of the second strand that is performed in the current methods. In the methods provided herein, the extended capture probe is removed from a spatially-barcoded array immediately after reverse transcription. The methods provided herein avoid the use of the reagents and enzymatic reactions that are necessary to generate and release the second strand. The methods provided herein can, e.g., increase sensitivity due to the second strand potentially not being efficiently generated for all extended capture probes attached to the spatially-barcoded array.

The methods provided herein utilize a combination of a detergent and a base, and optionally heat, to release the extended capture probe from a spatially-barcoded array. In general, the methods provided herein require, after production of an extended capture probe, incubating the spatially-barcoded array with a base and a detergent, and optionally, heating the spatially-barcoded array for a period of time, to release the extended capture probe from the spatially-barcoded array. Following this step, the released extended capture probe can be transferred to a container and neutralized, and subsequently amplified for library construction and sequencing. This method has the advatange of reduced processing time and a reduction in required sample processing materials.

Provided herein are methods of determining a location of a target analyte in a biological sample, the method includes: (a) contacting the biological sample with an array includes a plurality of capture probes, where a capture probe of the plurality includes (i) a capture domain and (ii) a spatial barcode; (b) releasing one or more target analyte(s) from the biological sample, where a target analyte of the one or more target analyte(s) that is released from the biological sample is hybridized by the capture domain of the capture probe; (c) extending an end of the capture probe using the target analyte hybridized to the capture domain of the capture probe as a template, to generate an extended capture probe; (d) exposing the capture probe to: (i) a base; and (ii) a detergent, where the exposing results in release of the extended capture probe of step (c) from the array; (e) adding a neutralizing agent; and (f) determining (i) all or a part of a sequence corresponding to the target analyte hybridized to the capture domain or a complement thereof, and (ii) all of a sequence corresponding to the spatial barcode or complement thereof, and using the determined sequences of (i) and (ii) to determine the location of the target analyte in the biological sample.

In some embodiments, the method includes, between steps (d) and (e), disposing the released extended capture probe into a receptacle, and step (e) includes adding the neutralizing agent to the receptacle.

In some embodiments, in step (a), the capture domain is positioned at a 3' end of the capture probe. In some embodiments, the capture probe includes a unique molecular identifier (UMI) and the UMI is positioned 5' relative to the capture domain.

In some embodiments, step (c) includes extending a 3' end of the capture probe using the target analyte hybridized to the capture domain of the capture probe as a template, to generate the extended capture probe. In some embodiments, step (c) includes the use of a reverse transcriptase.

In some embodiments, the detergent is a non-ionic detergent. In some embodiments, the non-ionic detergent is Triton-X 100. In some embodiments, the detergent is an anionic detergent. In some embodiments, the anionic detergent is sodium dodecyl sulfate (SDS). In some embodiments, the detergent is present at a concentration of about 0.1% w/v to about 2.0% w/v.

In some embodiments, the base is potassium hydroxide. In some embodiments, the base is present at a concentration of about 0.01 M to about 0.3 M. In some embodiments, the exposing is performed at a temperature of about 30 °C to about 80 °C. In some embodiments, the exposing is performed for about 1 minute to about 2 hours. In some embodiments, the neutralizing agent is an acid. In some embodiments, the neutralizing agent is a buffer. In some embodiments, the buffer is 2-amino-2-(hydroxymethyl) propane-1,3-diol or 2-[4-(2-hydroxyethyl)piperazin-1-yl]ethanesulfonic acid.

In some embodiments, the determining in step (f) includes sequencing (i) all or a part of the sequence corresponding to the target analyte specifically hybridized to the capture domain or the complement thereof, and (ii) all or a part of the sequence corresponding to the spatial barcode or the complement thereof. In some embodiments, the sequencing is high throughput sequencing.

In some embodiments, the target analyte is an RNA or an mRNA. In some embodiments, the capture domain includes a poly(T) sequence. In some embodiments, the target analyte is DNA or genomic DNA.

In some embodiments, the biological sample is a tissue sample, a tissue section or a fixed tissue section, and optionally where the fixed tissue section is a formalin-fixed paraffin-embedded tissue section or the tissue section is a fresh, frozen tissue section.

In some embodiments, where the determining in step (f) includes: amplifying an extended capture probe to generate an amplification product. In some embodiments, the determining in step (f) includes: generating a library using the amplification product.

Also provided herein are kits includes a substrate includes a base, a detergent, and a substrate includes a plurality of capture probes, where a capture probe of the plurality includes a capture domain.

In some embodiments, the capture domain is positioned at a 3' end of the capture probe.

In some embodiments, the kit includes a reverse transcriptase.

In some embodiments, the detergent is a non-ionic detergent. In some embodiments, the non-ionic detergent is Triton-X 100. In some embodiments, the detergent is an anionic detergent. In some embodiments, the anionic detergent is sodium dodecyl sulfate (SDS). In some embodiments, the detergent is present at a concentration of about 0.1% w/v to about 2.0% w/v. In some embodiments, the detergent is present at a concentration of about 0.5% w/v to about 1.5% w/v. In some embodiments, the base is potassium hydroxide. In some embodiments, the base is present at a concentration of about 0.01 M to about 0.3 M. In some embodiments, the base is present at a concentration of about 0.05 M to about 0.15 M. In some embodiments, the kit includes a neutralizing agent. In some embodiments, the neutralizing agent is an acid. In some embodiments, the neutralizing agent is a buffer. In some embodiments, the buffer is 2-amino-2-(hydroxymethyl) propane-1,3-diol or 2-[4-(2-hydroxyethyl)piperazin-1-yl]ethanesulfonic acid.

In some embodiments, the kit includes instructions for performing any of the methods described herein.

All publications, patents, patent applications, and information available on the internet and mentioned in this specification are herein incorporated by reference to the same extent as if each individual publication, patent, patent application, or item of information was specifically and individually indicated to be incorporated by reference. To the extent publications, patents, patent applications, and items of information incorporated by reference contradict the disclosure contained in the specification, the specification is intended to supersede and/or take precedence over any such contradictory material.

Where values are described in terms of ranges, it should be understood that the description includes the disclosure of all possible sub-ranges within such ranges, as well as specific numerical values that fall within such ranges irrespective of whether a specific numerical value or specific sub-range is expressly stated.

The term "each," when used in reference to a collection of items, is intended to identify an individual item in the collection but does not necessarily refer to every item in the collection, unless expressly stated otherwise, or unless the context of the usage clearly indicates otherwise.

Various embodiments of the features of this disclosure are described herein. However, it should be understood that such embodiments are provided merely by way of example, and numerous variations, changes, and substitutions can occur to those skilled in the art without departing from the scope of this disclosure. It should also be understood that various alternatives to the specific embodiments described herein are also within the scope of this disclosure.

### DESCRIPTION OF DRAWINGS

The following drawings illustrate certain embodiments of the features and advantages of this disclosure. These embodiments are not intended to limit the scope of the appended claims in any manner. Like reference symbols in the drawings indicate like elements.
**FIG. 1** is a schematic diagram showing an example of a barcoded capture probe, as described herein.
**FIG. 2** is a schematic illustrating a cleavable capture probe, wherein the cleaved capture probe can enter into a non-permeabilized cell and bind to target analytes within the sample.
**FIG. 3** is a schematic diagram of an exemplary multiplexed spatially-barcoded feature.
**FIG. 4** is a schematic diagram of an exemplary analyte capture agent.
**FIG. 5** is a schematic diagram depicting an exemplary interaction between a feature-immobilized capture probe **524** and an analyte capture agent **526.**
**FIGs. 6A****,** **6B****,** and **6C** are schematics illustrating how streptavidin cell tags can be utilized in an array-based system to produce spatially-barcoded cells or cellular contents.
**FIG. 7A** shows a schematic of a capture probe bound with a target analyte immobilized on a slide.
**FIG. 7B** shows a schematic of an extended capture probe bound with a target analyte.
**FIG. 7C** shows a schematic of a base and a detergent being applied to the extended capture probe bound with a target analyte.
**FIG. 7D** shows a schematic of the extended capture probe bound with a target analyte released from the slide.
**FIG. 8A** shows an image depicting the fluorescence on an array substrate after release of extended capture probes using control conditions.
**FIG. 8B** shows an image depicting the fluorescence on an array substrate after release of extended capture probes after incubation with a buffer comprising 0.1 N KOH and 1 % w/v SDS at room temperature.
**FIG. 8C** shows an image depicting the fluorescence on an array substrate after release of extended capture probes after incubation with a buffer comprising 0.1 N KOH at room temperature.
**FIG. 8D** shows an image depicting the fluorescence on an array substrate after release of extended capture probes after incubation with a buffer comprising 1% w/v SDS at room temperature.
**FIG. 8E** shows an image depicting the fluorescence on an array substrate after release of extended capture probes after incubation with a buffer comprising 0.1 N KOH and 1 % w/v SDS at 50 °C.
**FIG. 8F** shows an image depicting the fluorescence on an array substrate after release of extended capture probes after incubation with a buffer comprising 0.1 N KOH at 50 °C.
**FIG. 9A** shows a spatially-resolved gene expression heat map of a mouse brain section generated using methods that generate a second strand using the use of a template-switching oligonucleotide.
**FIG. 9B** shows a spatially-resolved gene expression heat map of a mouse brain section generated using the methods described herein that release the extended capture probe from the array using a base and a detergent.
**FIG. 10** shows a table of outcomes in control and test conditions for four replicates.
**FIG. 11A** shows a plot of sequencing reads mapped confidently to the transcriptome targets in control conditions compared to test conditions.
**FIG. 11B** shows a plot of the fraction of usable reads in control conditions compared to test conditions.
**FIG. 11C** shows a plot of the fraction of reads containing a switch oligo sequence in control conditions compared to test conditions.
**FIG. 11D** shows a plot of the fraction of reads with either a primer or homopolymer sequence in control conditions compared to test conditions.
**FIG. 11E** shows a plot of median gene reads per spot in mouse control conditions compared to mouse test conditions.
**FIG. 11F** shows a plot of median UMI counts per spot in mouse control conditions compared to mouse test conditions.
**FIG. 12A** shows two images depicting gene expression UMI heat maps related to the release of extended capture probes in two mouse brain tissue replicates using control conditions.
**FIG. 12B** shows two images depicting gene clustering patterns of a mouse brain section in two replicates, matched to the same samples from **FIG. 12A****.**
**FIG. 12C** shows two images depicting gene expression UMI heat maps related to the release of extended capture probes in two mouse brain tissue replicates using KOH stripping conditions.
**FIG. 12D** shows two images depicting gene clustering patterns of a mouse brain section in two replicates, matched to the same samples from **FIG. 12C****.**
**FIG. 13** shows two ISH images identifying hippocalcin gene expression using bright-field imaging (left) or fluorescence fluorescence detection (right).
**FIG. 14A** shows replicate images of spatially-resolved hippocalcin gene expression UMI heat maps of mouse brain sections generated using control methods.
**FIG. 14B** shows replicate images of spatially-resolved hippocalcin gene expression UMI heat maps of mouse brain sections generated using methods that release the extended capture probe from the array using a KOH base.
**FIG. 15** shows shows two ISH images identifying protein kinase c alpha gene expression using using bright-field imaging (left), or fluorescence detection (right).
**FIG. 16A** shows replicate images of spatially-resolved protein kinase c alpha gene expression UMI heat maps of mouse brain sections generated using control methods.
**FIG. 16B** shows replicate images of spatially-resolved protein kinase c alpha gene expression UMI heat maps of mouse brain sections generated using methods that release the extended capture probe from the array using a KOH base.
**FIG. 17** shows a table of outcomes in control and KOH test conditions for four replicates as imaged in **FIG. 16A** and **FIG. 16B****.**
**FIG. 18** shows a table of outcomes in control and KOH test conditions for four replicates as imaged in **FIG. 16A** and **FIG. 16B****.**

### DETAILED DESCRIPTION

Spatial analysis methodologies and compositions described herein can provide a vast amount of analyte and/or expression data for a variety of analytes within a biological sample at high spatial resolution, while retaining native spatial context. Spatial analysis methods and compositions can include, e.g., the use of a capture probe including a spatial barcode (e.g., a nucleic acid sequence that provides information as to the location or position of an analyte within a cell or a tissue sample (e.g., mammalian cell or a mammalian tissue sample) and a capture domain that is capable of binding to an analyte (e.g., a protein and/or a nucleic acid) produced by and/or present in a cell. Spatial analysis methods and compositions can also include the use of a capture probe having a capture domain that captures an intermediate agent for indirect detection of an analyte. For example, the intermediate agent can include a nucleic acid sequence (e.g., a barcode) associated with the intermediate agent. Detection of the intermediate agent is therefore indicative of the analyte in the cell or tissue sample.

Non-limiting aspects of spatial analysis methodologies and compositions are described in U.S. Patent Nos. 10,774,374, 10,724,078, 10,480,022, 10,059,990, 10,041,949, 10,002,316, 9,879,313, 9,783,841, 9,727,810, 9,593,365, 8,951,726, 8,604,182, 7,709,198, U.S. Patent Application Publication Nos. 2020/239946, 2020/080136, 2020/0277663 , 2020/024641, 2019/330617, 2019/264268, 2020/256867, 2020/224244, 2019/194709, 2019/161796, 2019/085383, 2019/055594, 2018/216161, 2018/051322, 2018/0245142, 2017/241911, 2017/089811, 2017/067096, 2017/029875, 2017/0016053, 2016/108458, 2015/000854, 2013/171621, WO 2018/091676, WO 2020/176788, Rodriques et al., Science 363(6434):1463-1467, 2019; Lee et al., Nat. Protoc. 10(3):442-458, 2015; Trejo et al., PLoS ONE 14(2):e0212031, 2019; Chen et al., Science 348(6233):aaa6090, 2015; Gao et al., BMC Biol. 15:50, 2017; and Gupta et al., Nature Biotechnol. 36:1197-1202, 2018; the Visium

Spatial Gene Expression Reagent Kits User Guide (e.g., Rev C, dated June 2020), and/or the Visium Spatial Tissue Optimization Reagent Kits User Guide (e.g., Rev C, dated July 2020), both of which are available at the 10× Genomics Support Documentation website, and can be used herein in any combination, and each of which is incorporated herein by reference in their entireties. Further non-limiting aspects of spatial analysis methodologies and compositions are described herein.

Some general terminology that may be used in this disclosure can be found in Section (I)(b) of WO 2020/176788 and/or U.S. Patent Application Publication No. 2020/0277663. Typically, a "barcode" is a label, or identifier, that conveys or is capable of conveying information (e.g., information about an analyte in a sample, a bead, and/or a capture probe). A barcode can be part of an analyte, or independent of an analyte. A barcode can be attached to an analyte. A particular barcode can be unique relative to other barcodes. For the purpose of this disclosure, an "analyte" can include any biological substance, structure, moiety, or component to be analyzed. The term "target" can similarly refer to an analyte of interest.

Analytes can be broadly classified into one of two groups: nucleic acid analytes, and non-nucleic acid analytes. Examples of non-nucleic acid analytes include, but are not limited to, lipids, carbohydrates, peptides, proteins, glycoproteins (N-linked or O-linked), lipoproteins, phosphoproteins, specific phosphorylated or acetylated variants of proteins, amidation variants of proteins, hydroxylation variants of proteins, methylation variants of proteins, ubiquitylation variants of proteins, sulfation variants of proteins, viral proteins (e.g., viral capsid, viral envelope, viral coat, viral accessory, viral glycoproteins, viral spike, etc.), extracellular and intracellular proteins, antibodies, and antigen binding fragments. In some embodiments, the analyte(s) can be localized to subcellular location(s), including, for example, organelles, e.g., mitochondria, Golgi apparatus, endoplasmic reticulum, chloroplasts, endocytic vesicles, exocytic vesicles, vacuoles, lysosomes, etc. In some embodiments, analyte(s) can be peptides or proteins, including without limitation antibodies and enzymes. Additional examples of analytes can be found in Section (I)(c) of WO 2020/176788 and/or U.S. Patent Application Publication No. 2020/0277663. In some embodiments, an analyte can be detected indirectly, such as through detection of an intermediate agent, for example, a ligation product or an analyte capture agent (e.g., an oligonucleotide-conjugated antibody), such as those described herein.

A "biological sample" is typically obtained from the subject for analysis using any of a variety of techniques including, but not limited to, biopsy, surgery, and laser capture microscopy (LCM), and generally includes cells and/or other biological material from the subject. In some embodiments, a biological sample can be a tissue section. In some embodiments, a biological sample can be a fixed and/or stained biological sample (e.g., a fixed and/or stained tissue section). Non-limiting examples of stains include histological stains (e.g., hematoxylin and/or eosin) and immunological stains (e.g., fluorescent stains). In some embodiments, a biological sample (e.g., a fixed and/or stained biological sample) can be imaged. Biological samples are also described in Section (I)(d) of WO 2020/176788 and/or U.S. Patent Application Publication No. 2020/0277663.

In some embodiments, a biological sample is permeabilized with one or more permeabilization reagents. For example, permeabilization of a biological sample can facilitate analyte capture. Exemplary permeabilization agents and conditions are described in Section (I)(d)(ii)(13) or the Exemplary Embodiments Section of WO 2020/176788 and/or U.S. Patent Application Publication No. 2020/0277663.

Array-based spatial analysis methods involve the transfer of one or more analytes from a biological sample to an array of features on a substrate, where each feature is associated with a unique spatial location on the array. Subsequent analysis of the transferred analytes includes determining the identity of the analytes and the spatial location of the analytes within the biological sample. The spatial location of an analyte within the biological sample is determined based on the feature to which the analyte is bound (e.g., directly or indirectly) on the array, and the feature's relative spatial location within the array.

A "capture probe" refers to any molecule capable of capturing (directly or indirectly) and/or labelling an analyte (e.g., an analyte of interest) in a biological sample. In some embodiments, the capture probe is a nucleic acid or a polypeptide. In some embodiments, the capture probe includes a barcode (e.g., a spatial barcode and/or a unique molecular identifier (UMI)) and a capture domain). In some embodiments, a capture probe can include a cleavage domain and/or a functional domain (e.g., a primer-binding site, such as for next-generation sequencing (NGS)). See, e.g., Section (II)(b) (e.g., subsections (i)-(vi)) of WO 2020/176788 and/or U.S. Patent Application Publication No. 2020/0277663. Generation of capture probes can be achieved by any appropriate method, including those described in Section (II)(d)(ii) of WO 2020/176788 and/or U.S. Patent Application Publication No. 2020/0277663.

In some embodiments, more than one analyte type (e.g., nucleic acids and proteins) from a biological sample can be detected (e.g., simultaneously or sequentially) using any appropriate multiplexing technique, such as those described in Section (IV) of WO 2020/176788 and/or U.S. Patent Application Publication No. 2020/0277663.

In some embodiments, detection of one or more analytes (e.g., protein analytes) can be performed using one or more analyte capture agents. As used herein, an "analyte capture agent" refers to an agent that interacts with an analyte (e.g., an analyte in a biological sample) and with a capture probe (e.g., a capture probe attached to a substrate or a feature) to identify the analyte. In some embodiments, the analyte capture agent includes: (i) an analyte binding moiety (e.g., that binds to an analyte), for example, an antibody or antigen-binding fragment thereof; (ii) analyte binding moiety barcode; and (iii) an analyte capture sequence. As used herein, the term "analyte binding moiety barcode" refers to a barcode that is associated with or otherwise identifies the analyte binding moiety. As used herein, the term "analyte capture sequence" refers to a region or moiety configured to hybridize to, bind to, couple to, or otherwise interact with a capture domain of a capture probe. In some cases, an analyte binding moiety barcode (or portion thereof) may be able to be removed (e.g., cleaved) from the analyte capture agent. Additional description of analyte capture agents can be found in Section (II)(b)(ix) of WO 2020/176788 and/or Section (II)(b)(viii) U.S. Patent Application Publication No. 2020/0277663.

There are at least two methods to associate a spatial barcode with one or more neighboring cells, such that the spatial barcode identifies the one or more cells, and/or contents of the one or more cells, as associated with a particular spatial location. One method is to promote analytes or analyte proxies (e.g., intermediate agents) out of a cell and towards a spatially-barcoded array (e.g., including spatially-barcoded capture probes). Another method is to cleave spatially-barcoded capture probes from an array and promote the spatially-barcoded capture probes towards and/or into or onto the biological sample.

In some cases, capture probes may be configured to prime, replicate, and consequently yield optionally barcoded extension products from a template (e.g., a DNA or RNA template, such as an analyte or an intermediate agent (e.g., a ligation product or an analyte capture agent), or a portion thereof), or derivatives thereof (see, e.g., Section (II)(b)(vii) of WO 2020/176788 and/or U.S. Patent Application Publication No. 2020/0277663 regarding extended capture probes). In some cases, capture probes may be configured to form ligation products with a template (e.g., a DNA or RNA template, such as an analyte or an intermediate agent, or portion thereof), thereby creating ligations products that serve as proxies for a template.

As used herein, an "extended capture probe" refers to a capture probe having additional nucleotides added to the terminus (e.g., 3' or 5' end) of the capture probe thereby extending the overall length of the capture probe. For example, an "extended 3' end" indicates additional nucleotides were added to the most 3' nucleotide of the capture probe to extend the length of the capture probe, for example, by polymerization reactions used to extend nucleic acid molecules including templated polymerization catalyzed by a polymerase (e.g., a DNA polymerase or a reverse transcriptase). In some embodiments, extending the capture probe includes adding to a 3' end of a capture probe a nucleic acid sequence that is complementary to a nucleic acid sequence of an analyte or intermediate agent specifically bound to the capture domain of the capture probe. In some embodiments, the capture probe is extended using reverse transcription. In some embodiments, the capture probe is extended using one or more DNA polymerases. The extended capture probes include the sequence of the capture probe and the sequence of the spatial barcode of the capture probe.

In some embodiments, extended capture probes are amplified (e.g., in bulk solution or on the array) to yield quantities that are sufficient for downstream analysis, e.g., via DNA sequencing. In some embodiments, extended capture probes (e.g., DNA molecules) act as templates for an amplification reaction (e.g., a polymerase chain reaction).

Additional variants of spatial analysis methods, including in some embodiments, an imaging step, are described in Section (II)(a) of WO 2020/176788 and/or U.S. Patent Application Publication No. 2020/0277663. Analysis of captured analytes (and/or intermediate agents or portions thereof), for example, including sample removal, extension of capture probes, sequencing (e.g., of a cleaved extended capture probe and/or a cDNA molecule complementary to an extended capture probe), sequencing on the array (e.g., using, for example, in situ hybridization or in situ ligation approaches), temporal analysis, and/or proximity capture, is described in Section (II)(g) of WO 2020/176788 and/or U.S. Patent Application Publication No. 2020/0277663. Some quality control measures are described in Section (II)(h) of WO 2020/176788 and/or U.S. Patent Application Publication No. 2020/0277663.

Spatial information can provide information of biological and/or medical importance. For example, the methods and compositions described herein can allow for: identification of one or more biomarkers (e.g., diagnostic, prognostic, and/or for determination of efficacy of a treatment) of a disease or disorder; identification of a candidate drug target for treatment of a disease or disorder; identification (e.g., diagnosis) of a subject as having a disease or disorder; identification of stage and/or prognosis of a disease or disorder in a subject; identification of a subject as having an increased likelihood of developing a disease or disorder; monitoring of progression of a disease or disorder in a subject; determination of efficacy of a treatment of a disease or disorder in a subj ect; identification of a patient subpopulation for which a treatment is effective for a disease or disorder; modification of a treatment of a subject with a disease or disorder; selection of a subject for participation in a clinical trial; and/or selection of a treatment for a subject with a disease or disorder. Exemplary methods for identifying spatial information of biological and/or medical importance can be found in U.S. Patent Application Publication No. 2021/0140982A1, U.S.

Patent Application No. 2021/0198741A1, and/or U.S. Patent Application No. 2021/0199660.

Spatial information can provide information of biological importance. For example, the methods and compositions described herein can allow for: identification of transcriptome and/or proteome expression profiles (e.g., in healthy and/or diseased tissue); identification of multiple analyte types in close proximity (e.g., nearest neighbor analysis); determination of up- and/or down-regulated genes and/or proteins in diseased tissue; characterization of tumor microenvironments; characterization of tumor immune responses; characterization of cells types and their co-localization in tissue; and identification of genetic variants within tissues (e.g., based on gene and/or protein expression profiles associated with specific disease or disorder biomarkers).

Typically, for spatial array-based methods, a substrate functions as a support for direct or indirect attachment of capture probes to features of the array. A "feature" is an entity that acts as a support or repository for various molecular entities used in spatial analysis. In some embodiments, some or all of the features in an array are functionalized for analyte capture. Exemplary substrates are described in Section (II)(c) of WO 2020/176788 and/or U.S. Patent Application Publication No. 2020/0277663. Exemplary features and geometric attributes of an array can be found in Sections (II)(d)(i), (II)(d)(iii), and (II)(d)(iv) of WO 2020/176788 and/or U.S. Patent Application Publication No. 2020/0277663.

Generally, analytes and/or intermediate agents (or portions thereof) can be captured when contacting a biological sample with a substrate including capture probes (e.g., a substrate with capture probes embedded, spotted, printed, fabricated on the substrate, or a substrate with features (e.g., beads, wells) comprising capture probes). As used herein, "contact," "contacted," and/or "contacting," a biological sample with a substrate refers to any contact (e.g., direct or indirect) such that capture probes can interact (e.g., bind covalently or non-covalently (e.g., hybridize)) with analytes from the biological sample. Capture can be achieved actively (e.g., using electrophoresis) or passively (e.g., using diffusion). Analyte capture is further described in Section (II)(e) of WO 2020/176788 and/or U.S. Patent Application Publication No. 2020/0277663.

In some cases, spatial analysis can be performed by attaching and/or introducing a molecule (e.g., a peptide, a lipid, or a nucleic acid molecule) having a barcode (e.g., a spatial barcode) to a biological sample (e.g., to a cell in a biological sample). In some embodiments, a plurality of molecules (e.g., a plurality of nucleic acid molecules) having a plurality of barcodes (e.g., a plurality of spatial barcodes) are introduced to a biological sample (e.g., to a plurality of cells in a biological sample) for use in spatial analysis. In some embodiments, after attaching and/or introducing a molecule having a barcode to a biological sample, the biological sample can be physically separated (e.g., dissociated) into single cells or cell groups for analysis. Some such methods of spatial analysis are described in Section (III) of WO 2020/176788 and/or U.S. Patent Application Publication No. 2020/0277663.

In some cases, spatial analysis can be performed by detecting multiple oligonucleotides that hybridize to an analyte. In some instances, for example, spatial analysis can be performed using RNA-templated ligation (RTL). Methods of RTL have been described previously. See, e.g., Credle et al., Nucleic Acids Res. 2017 Aug 21; 45(14):e128. Typically, RTL includes hybridization of two oligonucleotides to adjacent sequences on an analyte (e.g., an RNA molecule, such as an mRNA molecule). In some instances, the oligonucleotides are DNA molecules. In some instances, one of the oligonucleotides includes at least two ribonucleic acid bases at the 3' end and/or the other oligonucleotide includes a phosphorylated nucleotide at the 5' end. In some instances, one of the two oligonucleotides includes a capture domain (e.g., a poly(A) sequence, a non-homopolymeric sequence). After hybridization to the analyte, a ligase (e.g., SplintR ligase) ligates the two oligonucleotides together, creating a ligation product. In some instances, the two oligonucleotides hybridize to sequences that are not adjacent to one another. For example, hybridization of the two oligonucleotides creates a gap between the hybridized oligonucleotides. In some instances, a polymerase (e.g., a DNA polymerase) can extend one of the oligonucleotides prior to ligation. After ligation, the ligation product is released from the analyte. In some instances, the ligation product is released using an endonuclease (e.g., RNAse H). The released ligation product can then be captured by capture probes (e.g., instead of direct capture of an analyte) on an array, optionally amplified, and sequenced, thus determining the location and optionally the abundance of the analyte in the biological sample.

During analysis of spatial information, sequence information for a spatial barcode associated with an analyte is obtained, and the sequence information can be used to provide information about the spatial distribution of the analyte in the biological sample. Various methods can be used to obtain the spatial information. In some embodiments, specific capture probes and the analytes they capture are associated with specific locations in an array of features on a substrate. For example, specific spatial barcodes can be associated with specific array locations prior to array fabrication, and the sequences of the spatial barcodes can be stored (e.g., in a database) along with specific array location information, so that each spatial barcode uniquely maps to a particular array location.

Alternatively, specific spatial barcodes can be deposited at predetermined locations in an array of features during fabrication such that at each location, only one type of spatial barcode is present so that spatial barcodes are uniquely associated with a single feature of the array. Where necessary, the arrays can be decoded using any of the methods described herein so that spatial barcodes are uniquely associated with array feature locations, and this mapping can be stored as described above.

When sequence information is obtained for capture probes and/or analytes during analysis of spatial information, the locations of the capture probes and/or analytes can be determined by referring to the stored information that uniquely associates each spatial barcode with an array feature location. In this manner, specific capture probes and captured analytes are associated with specific locations in the array of features. Each array feature location represents a position relative to a coordinate reference point (e.g., an array location, a fiducial marker) for the array. Accordingly, each feature location has an "address" or location in the coordinate space of the array.

Some exemplary spatial analysis workflows are described in the Exemplary Embodiments section of WO 2020/176788 and/or U.S. Patent Application Publication No. 2020/0277663. See, for example, the Exemplary embodiment starting with "In some non-limiting examples of the workflows described herein, the sample can be immersed... " of WO 2020/176788 and/or U.S. Patent Application Publication No. 2020/0277663. See also, e.g., the Visium Spatial Gene Expression Reagent Kits User Guide (e.g., Rev C, dated June 2020), and/or the Visium Spatial Tissue Optimization Reagent Kits User Guide (e.g., Rev C, dated July 2020).

In some embodiments, spatial analysis can be performed using dedicated hardware and/or software, such as any of the systems described in Sections (II)(e)(ii) and/or (V) of WO 2020/176788 and/or U.S. Patent Application Publication No. 2020/0277663, or any of one or more of the devices or methods described in Sections Control Slide for Imaging, Methods of Using Control Slides and Substrates for, Systems of Using Control Slides and Substrates for Imaging, and/or Sample and Array Alignment Devices and Methods, Informational labels of WO 2020/123320.

Suitable systems for performing spatial analysis can include components such as a chamber (e.g., a flow cell or sealable, fluid-tight chamber) for containing a biological sample. The biological sample can be mounted for example, in a biological sample holder. One or more fluid chambers can be connected to the chamber and/or the sample holder via fluid conduits, and fluids can be delivered into the chamber and/or sample holder via fluidic pumps, vacuum sources, or other devices coupled to the fluid conduits that create a pressure gradient to drive fluid flow. One or more valves can also be connected to fluid conduits to regulate the flow of reagents from reservoirs to the chamber and/or sample holder.

The systems can optionally include a control unit that includes one or more electronic processors, an input interface, an output interface (such as a display), and a storage unit (e.g., a solid state storage medium such as, but not limited to, a magnetic, optical, or other solid state, persistent, writeable and/or re-writeable storage medium). The control unit can optionally be connected to one or more remote devices via a network. The control unit (and components thereof) can generally perform any of the steps and functions described herein. Where the system is connected to a remote device, the remote device (or devices) can perform any of the steps or features described herein. The systems can optionally include one or more detectors (e.g., CCD, CMOS) used to capture images. The systems can also optionally include one or more light sources (e.g., LED-based, diode-based, lasers) for illuminating a sample, a substrate with features, analytes from a biological sample captured on a substrate, and various control and calibration media.

The systems can optionally include software instructions encoded and/or implemented in one or more of tangible storage media and hardware components such as application specific integrated circuits. The software instructions, when executed by a control unit (and in particular, an electronic processor) or an integrated circuit, can cause the control unit, integrated circuit, or other component executing the software instructions to perform any of the method steps or functions described herein.

In some cases, the systems described herein can detect (e.g., register an image) the biological sample on the array. Exemplary methods to detect the biological sample on an array are described in WO 2021/102003 and/or U.S. Patent Application Serial No. 16/951,854, each of which is incorporated herein by reference in their entireties.

Prior to transferring analytes from the biological sample to the array of features on the substrate, the biological sample can be aligned with the array. Alignment of a biological sample and an array of features including capture probes can facilitate spatial analysis, which can be used to detect differences in analyte presence and/or level within different positions in the biological sample, for example, to generate a three-dimensional map of the analyte presence and/or level. Exemplary methods to generate a two- and/or three-dimensional map of the analyte presence and/or level are described in PCT Application No. 2020/053655 and spatial analysis methods are generally described in WO 2021/102039 and/or U.S. Patent Application Serial No. 16/951,864, each of which is incorporated herein by reference in their entireties.

In some cases, a map of analyte presence and/or level can be aligned to an image of a biological sample using one or more fiducial markers, e.g., objects placed in the field of view of an imaging system which appear in the image produced, as described in the Substrate Attributes Section, Control Slide for Imaging Section of WO 2020/123320, WO 2021/102005, and/or U.S. Patent Application Serial No. 16/951,843, each of which is incorporated herein by reference in their entireties. Fiducial markers can be used as a point of reference or measurement scale for alignment (e.g., to align a sample and an array, to align two substrates, to determine a location of a sample or array on a substrate relative to a fiducial marker) and/or for quantitative measurements of sizes and/or distances.

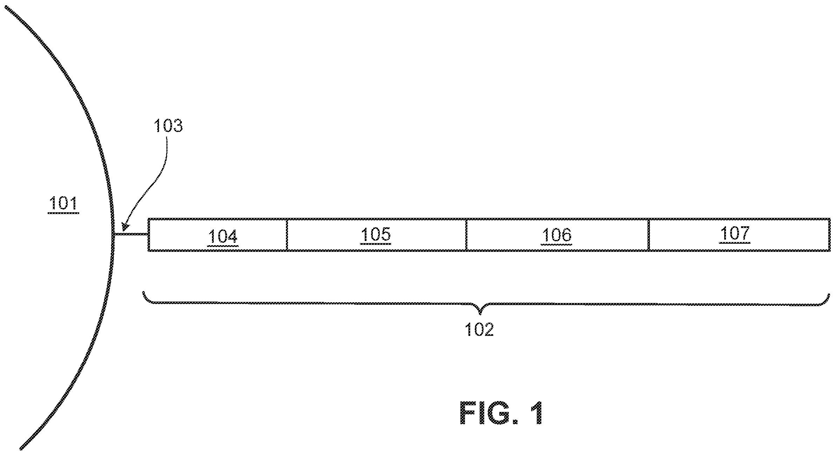

**FIG. 1** is a schematic diagram showing an exemplary capture probe, as described herein. As shown, the capture probe **102** is optionally coupled to a feature **101** by a cleavage domain **103,** such as a disulfide linker. The capture probe can include a functional sequence **104** that is useful for subsequent processing. The functional sequence **104** can include all or a part of sequencer specific flow cell attachment sequence (e.g., a P5 or P7 sequence), all or a part of a sequencing primer sequence, (e.g., a R1 primer binding site, a R2 primer binding site), or combinations thereof. The capture probe can also include a spatial barcode **105**. The capture probe can also include a unique molecular identifier (UMI) sequence **106.** While **FIG. 1** shows the spatial barcode **105** as being located upstream (5') of UMI sequence **106,** it is to be understood that capture probes wherein UMI sequence **106** is located upstream (5') of the spatial barcode **105** is also suitable for use in any of the methods described herein. The capture probe can also include a capture domain **107** to facilitate capture of a target analyte. The capture domain can have a sequence complementary to a sequence of a nucleic acid analyte. The capture domain can have a sequence complementary to a connected probe described herein. The capture domain can have a sequence complementary to a capture handle sequence present in an analyte capture agent. The capture domain can have a sequence complementary to a splint oligonucleotide. Such splint oligonucleotide, in addition to having a sequence complementary to a capture domain of a capture probe, can have a sequence of a nucleic acid analyte, a sequence complementary to a portion of a connected probe described herein, and/or a capture handle sequence described herein.

The functional sequences can generally be selected for compatibility with any of a variety of different sequencing systems, e.g., Ion Torrent Proton or PGM, Illumina sequencing instruments, PacBio, Oxford Nanopore, etc., and the requirements thereof. In some embodiments, functional sequences can be selected for compatibility with non-commercialized sequencing systems. Examples of such sequencing systems and techniques, for which suitable functional sequences can be used, include (but are not limited to) Ion Torrent Proton or PGM sequencing, Illumina sequencing, PacBio SMRT sequencing, and Oxford Nanopore sequencing. Further, in some embodiments, functional sequences can be selected for compatibility with other sequencing systems, including non-commercialized sequencing systems.

In some embodiments, the spatial barcode **105** and functional sequences **104** are common to all of the probes attached to a given feature. In some embodiments, the UMI sequence **106** of a capture probe attached to a given feature is different from the UMI sequence of a different capture probe attached to the given feature.

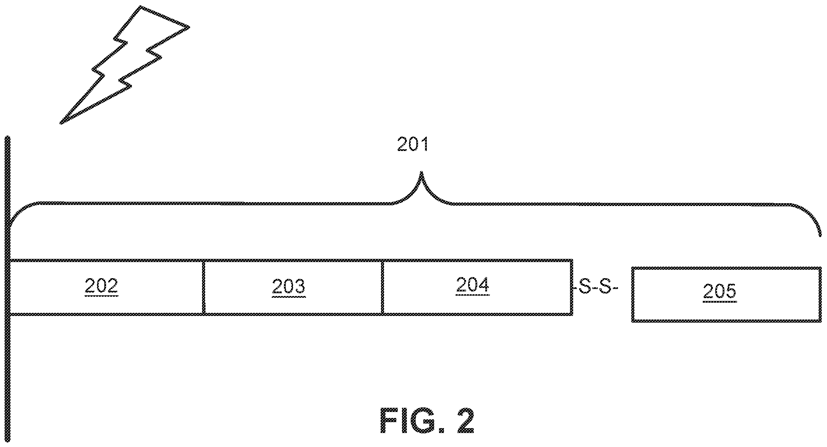

**FIG. 2** is a schematic illustrating a cleavable capture probe, wherein the cleaved capture probe can enter into a non-permeabilized cell and bind to analytes within the sample. The capture probe **201** contains a cleavage domain **202,** a cell penetrating peptide **203,** a reporter molecule **204,** and a disulfide bond (-S-S-). **205** represents all other parts of a capture probe, for example a spatial barcode and a capture domain.

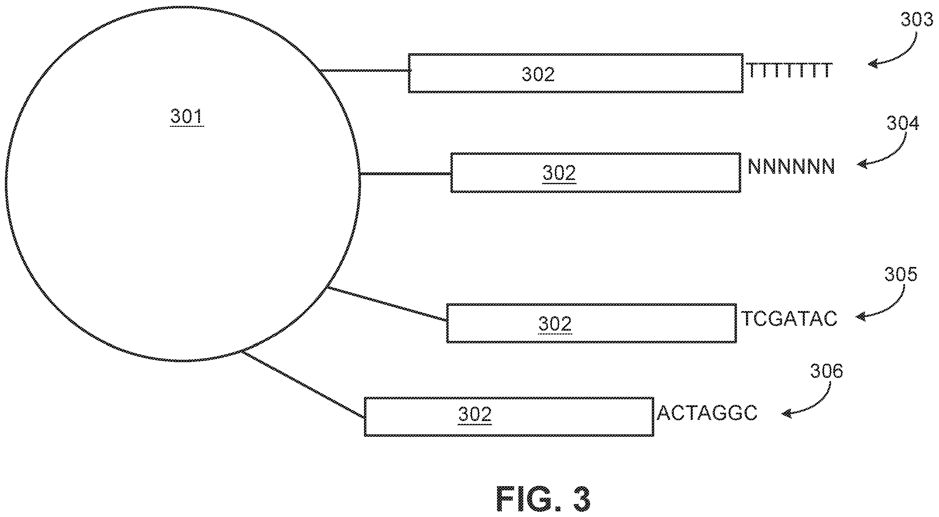

**FIG. 3** is a schematic diagram of an exemplary multiplexed spatially-barcoded feature. In **FIG. 3****,** the feature **301** can be coupled to spatially-barcoded capture probes, wherein the spatially-barcoded probes of a particular feature can possess the same spatial barcode, but have different capture domains designed to associate the spatial barcode of the feature with more than one target analyte. For example, a feature may be coupled to four different types of spatially-barcoded capture probes, each type of spatially-barcoded capture probe possessing the spatial barcode **302.** One type of capture probe associated with the feature includes the spatial barcode **302** in combination with a poly(T) capture domain **303,** designed to capture mRNA target analytes. A second type of capture probe associated with the feature includes the spatial barcode **302** in combination with a random N-mer capture domain **304** for gDNA analysis. A third type of capture probe associated with the feature includes the spatial barcode **302** in combination with a capture domain complementary to a capture handle sequence of an analyte capture agent of interest **305.** A fourth type of capture probe associated with the feature includes the spatial barcode **302** in combination with a capture domain that can specifically bind a nucleic acid molecule **306** that can function in a CRISPR assay (e.g., CRISPR/Cas9). While only four different capture probe-barcoded constructs are shown in **FIG. 3****,** capture-probe barcoded constructs can be tailored for analyses of any given analyte associated with a nucleic acid and capable of binding with such a construct. For example, the schemes shown in **FIG. 3** can also be used for concurrent analysis of other analytes disclosed herein, including, but not limited to: (a) mRNA, a lineage tracing construct, cell surface or intracellular proteins and metabolites, and gDNA; (b) mRNA, accessible chromatin (e.g., ATAC-seq, DNase-seq, and/or MNase-seq) cell surface or intracellular proteins and metabolites, and a perturbation agent (e.g., a CRISPR crRNA/sgRNA, TALEN, zinc finger nuclease, and/or antisense oligonucleotide as described herein); (c) mRNA, cell surface or intracellular proteins and/or metabolites, a barcoded labelling agent (e.g., the MHC multimers described herein), and a V(D)J sequence of an immune cell receptor (e.g., T-cell receptor). In some embodiments, a perturbation agent can be a small molecule, an antibody, a drug, an aptamer, a miRNA, a physical environmental (e.g., temperature change), or any other known perturbation agents. See, e.g., Section (II)(b) (e.g., subsections (i)-(vi)) of WO 2020/176788 and/or U.S. Patent Application Publication No. 2020/0277663. Generation of capture probes can be achieved by any appropriate method, including those described in Section (II)(d)(ii) of WO 2020/176788 and/or U.S. Patent Application Publication No. 2020/0277663.

In some embodiments, more than one analyte type (e.g., nucleic acids and proteins) from a biological sample can be detected (e.g., simultaneously or sequentially) using any appropriate multiplexing technique, such as those described in Section (IV) of WO 2020/176788 and/or U.S. Patent Application Publication No. 2020/0277663.

In some embodiments, detection of one or more analytes (e.g., protein analytes) can be performed using one or more analyte capture agents. As used herein, an "analyte capture agent" refers to an agent that interacts with an analyte (e.g., an analyte in a biological sample) and with a capture probe (e.g., a capture probe attached to a substrate or a feature) to identify the analyte. In some embodiments, the analyte capture agent includes: (i) an analyte binding moiety (e.g., that binds to an analyte), for example, an antibody or antigen-binding fragment thereof; (ii) analyte binding moiety barcode; and (iii) a capture handle sequence. As used herein, the term "analyte binding moiety barcode" refers to a barcode that is associated with or otherwise identifies the analyte binding moiety. As used herein, the term "analyte capture sequence" or "capture handle sequence" refers to a region or moiety configured to hybridize to, bind to, couple to, or otherwise interact with a capture domain of a capture probe. In some embodiments, a capture handle sequence is complementary to a capture domain of a capture probe. In some cases, an analyte binding moiety barcode (or portion thereof) may be able to be removed (e.g., cleaved) from the analyte capture agent.

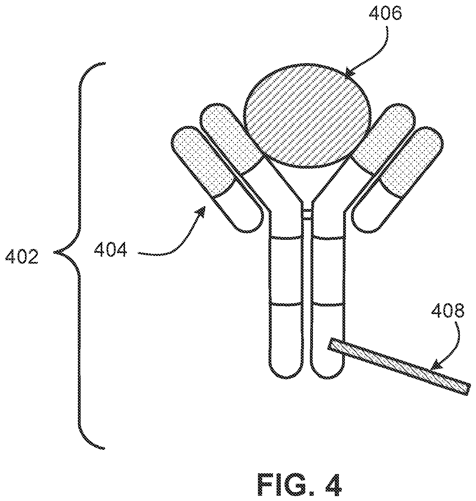

**FIG. 4** is a schematic diagram of an exemplary analyte capture agent **402** comprised of an analyte-binding moiety **404** and an analyte-binding moiety barcode domain **408**. The exemplary analyte -binding moiety **404** is a molecule capable of binding to an analyte **406** and the analyte capture agent is capable of interacting with a spatially-barcoded capture probe. The analyte -binding moiety can bind to the analyte **406** with high affinity and/or with high specificity. The analyte capture agent can include an analyte -binding moiety barcode domain **408,** a nucleotide sequence (e.g., an oligonucleotide), which can hybridize to at least a portion or an entirety of a capture domain of a capture probe. The analyte-binding moiety barcode domain **408** can comprise an analyte binding moiety barcode and a capture handle sequence described herein. The analyte -binding moiety **404** can include a polypeptide and/or an aptamer. The analyte -binding moiety **404** can include an antibody or antibody fragment (e.g., an antigen-binding fragment).

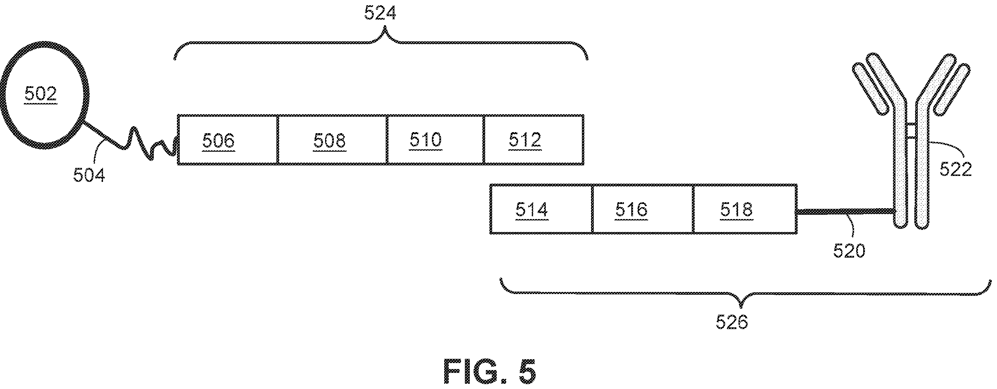

**FIG. 5** is a schematic diagram depicting an exemplary interaction between a feature-immobilized capture probe **524** and an analyte capture agent **526.** The feature-immobilized capture probe **524** can include a spatial barcode **508** as well as functional sequences **506** and UMI **510,** as described elsewhere herein. The capture probe can also include a capture domain **512** that is capable of binding to an analyte capture agent **526.** The analyte capture agent **526** can include a functional sequence **518,** analyte binding moiety barcode **516,** and a capture handle sequence **514** that is capable of binding to the capture domain **512** of the capture probe **524.** The analyte capture agent can also include a linker **520** that allows the capture agent barcode domain **516** to couple to the analyte binding moiety **522.**

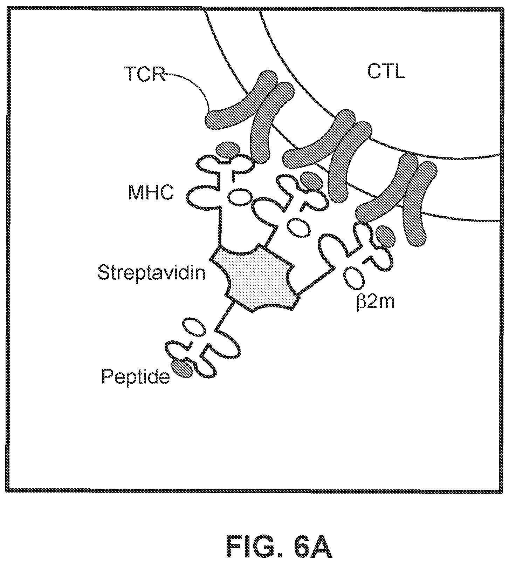

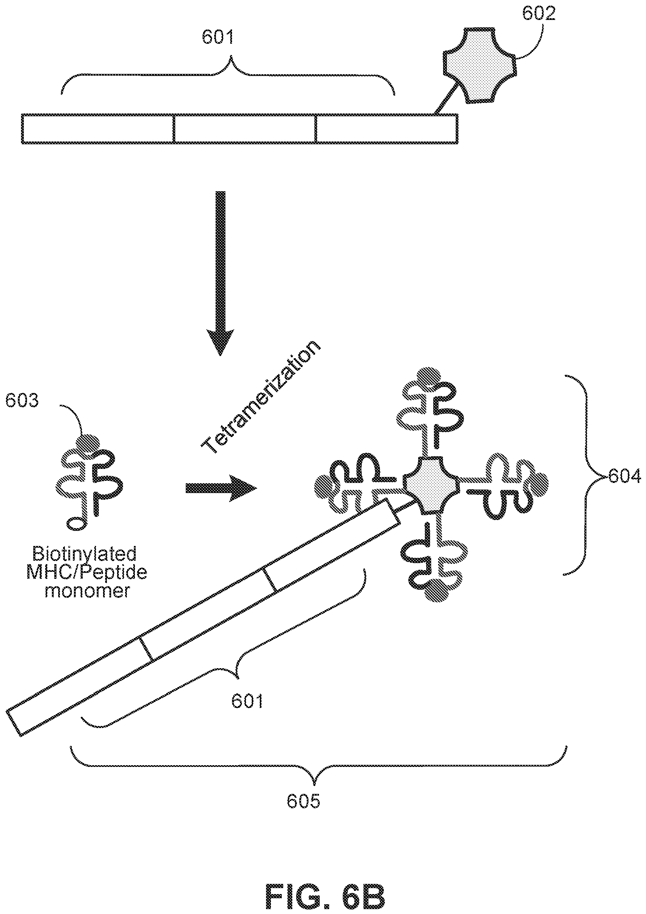

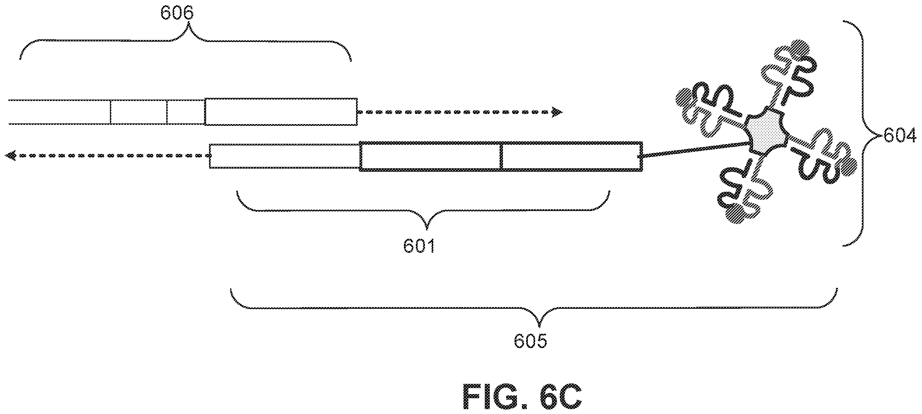

**FIGS. 6A****,** **6B****,** and **6C** are schematics illustrating how streptavidin cell tags can be utilized in an array-based system to produce a spatially-barcoded cell or cellular contents. For example, as shown in **FIG. 6A****,** peptide-bound major histocompatibility complex (MHC) can be individually associated with biotin (β2m) and bound to a streptavidin moiety such that the streptavidin moiety comprises multiple pMHC moieties. Each of these moieties can bind to a TCR such that the streptavidin binds to a target T-cell via multiple MHC/TCR binding interactions. Multiple interactions synergize and can substantially improve binding affinity. Such improved affinity can improve labelling of T-cells and also reduce the likelihood that labels will dissociate from T-cell surfaces. As shown in **FIG. 6B****,** a capture agent barcode domain **601** can be modified with streptavidin **602** and contacted with multiple molecules of biotinylated MHC **603** such that the biotinylated MHC **603** molecules are coupled with the streptavidin conjugated capture agent barcode domain **601.** The result is a barcoded MHC multimer complex **605.** As shown in **FIG. 6**B, the capture agent barcode domain sequence **601** can identify the MHC as its associated label and also includes optional functional sequences such as sequences for hybridization with other oligonucleotides. As shown in **FIG. 6C****,** one example oligonucleotide is capture probe **606** that comprises a complementary sequence (e.g., rGrGrG corresponding to C C C), a barcode sequence and other functional sequences, such as, for example, a UMI, an adapter sequence (e.g., comprising a sequencing primer sequence (e.g., R1 or a partial R1 ("pRF"), R2), a flow cell attachment sequence (e.g., P5 or P7 or partial sequences thereof)), etc. In some cases, capture probe **606** may at first be associated with a feature (e.g., a gel bead) and released from the feature. In other embodiments, capture probe **606** can hybridize with a capture agent barcode domain **601** of the MHC-oligonucleotide complex **605.** The hybridized oligonucleotides (Spacer C C C and Spacer rGrGrG) can then be extended in primer extension reactions such that constructs comprising sequences that correspond to each of the two spatial barcode sequences (the spatial barcode associated with the capture probe, and the barcode associated with the MHC-oligonucleotide complex) are generated. In some cases, one or both of the corresponding sequences may be a complement of the original sequence in capture probe **606** or capture agent barcode domain **601.** In other embodiments, the capture probe and the capture agent barcode domain are ligated together. The resulting constructs can be optionally further processed (e.g., to add any additional sequences and/or for clean-up) and subjected to sequencing. As described elsewhere herein, a sequence derived from the capture probe **606** spatial barcode sequence may be used to identify a feature and the sequence derived from spatial barcode sequence on the capture agent barcode domain **601** may be used to identify the particular peptide MHC complex **604** bound on the surface of the cell (e.g., when using MHC-peptide libraries for screening immune cells or immune cell populations).

### Methods of Releasing Extended Capture Probes

After the application of a biological sample on a spatially-barcoded array, the captured analytes are reverse transcribed to produce an extended capture probe (also called a first-strand cDNA). The extended capture probe comprises all the sequences of the capture probe and the complementary sequence of the captured analyte, for example a captured mRNA. In order to prepare a library, the captured analyte is removed, including by degradation or denaturation, from the extended capture probe and a double-stranded template switch oligonucleotide (TSO) is concatenated onto the 3' end of the extended capture probe. A polymerase enzyme is used to generate a second strand from the extended capture probe that includes the spatial barcode information and other capture probe sequences (e.g., a complement of the spatial barcode, UMI, functional sequence(s), etc.). This second strand is released from the extended capture probe of the spatially-barcoded array and transferred to a new tube for amplification and sequencing. This methodology requires additional enzyme, primer, and buffer in order to synthesize the second strand from the extended capture probe, thereby increasing time, cost, and the potential for inefficiencies while practicing the methods.

Provided herein are methods for releasing an extended capture probe from a spatially-barcoded array. These methods provide for a reduction in both the time and costs associated with the generation and release of the second strand cDNA in the current methods. Further, the methods provide for decreasing the potential of inefficient enzymatic reactions which could occur during second strand cDNA synthesis. In the methods provided herein, the extended capture probe, or first strand cDNA, is removed from a spatially-barcoded array immediately after reverse transcription. The methods provided herein avoid the use of the reagents and enzymatic reactions that are necessary to generate and release second strand cDNA. The methods provided herein can, e.g., increase sensitivity due to the second strand cDNA potentially not being efficiently generated for all extended capture probes attached to the spatially-barcoded array.

The methods provided herein utilize a combination of a detergent and a base, and optionally heat, to release an extended capture probe from a spatially-barcoded array. In general, the methods provided herein require, after production of an extended capture probe, incubating the spatially-barcoded array with a base and a detergent, and optionally, heating the spatially-barcoded array for a period of time, to release the extended capture probe from the spatially-barcoded array. Following release from the array, the extended capture probe can be transferred to a container, the base neutralized, and the released extended capture probe can be subsequently amplified for downstream applications such as library construction and sequencing. The disclosed methods have the advantage of reducing processing time, material costs, and any inefficiencies that might have occurred during the normal process of second strand cDNA synthesis.

Provided herein are methods that include the steps of: (a) contacting the biological sample (e.g., any of the exemplary biological samples described herein) with a substrate (e.g., any of the exemplary substrates described herein) comprising a plurality of capture probes (e.g., any of the exemplary capture probes described herein), where a capture probe of the plurality comprises a capture domain (e.g., any of the exemplary capture domains described herein); (b) releasing one or more target analyte(s) (e.g., any of the exemplary target analytes described herein) from the biological sample, where a target analyte of the one or more target analyte(s) that is released from the biological sample specifically hybridizes to the capture domain of the capture probe; (c) extending an end of the capture probe using the target analyte that is hybridized to the capture domain of the capture probe as a template to generate an extended capture probe; (d) exposing the capture probe to: (i) a base (e.g., any of the exemplary bases described herein); and (ii) a detergent (e.g., any of the exemplary detergents described herein), where the exposing results in release of the extended capture probe from the substrate.

Also provided herein are methods of determining a location of a target analyte (e.g., any of the exemplary target analytes described herein) in a biological sample (e.g., any of the exemplary biological samples described herein) that include: (a) contacting the biological sample with a substrate (e.g., any of the exemplary substrates described herein) comprising a plurality of capture probes (e.g., any of the exemplary capture probes described herein), where a capture probe of the plurality comprises a capture domain (e.g., any of the exemplary capture domains described herein) and a spatial barcode; (b) releasing one or more target analyte(s) from the biological sample, where a target analyte of the one or more target analyte(s) that is released from the biological sample is specifically bound by the capture domain of the capture probe; (c) extending an end of the capture probe using the target analyte that is specifically bound by the capture domain of the capture probe as a template, to generate an extended capture probe; (d) exposing the capture probe to: (i) a base (e.g., any of the exemplary bases described herein); and (ii) a detergent (e.g., any of the exemplary detergents described herein), where the exposing results in release of the extended capture probe of step (c) from the substrate; (e) adding a neutralizing agent (e.g., any of the exemplary neutralizing agents described herein); and (f) determining (i) all or a part of a sequence corresponding to the target analyte specifically bound by the capture domain or a complement thereof, and (ii) all or a part of a sequence corresponding to the spatial barcode or complement thereof, and using the determined sequences of (i) and (ii) to determine the location of the target analyte in the biological sample.

Also provided herein are methods that include: (a) contacting a biological sample (e.g., any of the exemplary biological samples described herein) with a plurality of analyte capture agents (e.g., any of the exemplary analyte capture agents described herein), where an analyte capture agent of the plurality of analyte capture agents comprises an analyte binding moiety (e.g., any of the exemplary analyte binding moieties described herein), an analyte binding moiety barcode, and an analyte capture sequence (e.g., any of the exemplary analyte capture sequences described herein); (b) contacting the biological sample with a substrate (e.g., any of the exemplary substrates described herein) comprising a plurality of capture probes (e.g., any of the exemplary capture probes described herein), where a capture probe of the plurality comprises a capture domain (e.g., any of the exemplary capture domains described herein) that binds specifically to the analyte capture sequence; (c) releasing one or more target analyte(s) (e.g., any of the exemplary target analytes described herein) from the biological sample, where a target analyte of the one or more target analyte(s) that is released from the biological sample is specifically bound by the analyte binding moiety of the analyte capture agent and the analyte capture sequence is specifically bound by the capture domain; (d) extending an end of the capture probe using the analyte capture sequence that is specifically bound by the capture domain of the capture probe as a template, to generate an extended capture probe; (e) exposing the capture probe to: (i) a base (e.g., any of the exemplary bases described herein); and (ii) a detergent (e.g., any of the exemplary detergents described herein), where the exposing results in release of the extended capture probe of step (d) from the substrate.

Also provided herein are methods of determining a location of a target analyte(s) (e.g., any of the exemplary target analytes described herein) in a biological sample (e.g., any of the exemplary biological samples described herein) that include: (a) contacting a biological sample with a plurality of analyte capture agents (e.g., any of the exemplary analyte capture agents described herein), where an analyte capture agent of the plurality of analyte capture agents comprises an analyte binding moiety (e.g., any of the analyte binding moieties described herein), analyte binding moiety barcode, and an analyte capture sequence (e.g., any of the exemplary analyte capture sequences described herein); (b) contacting the biological sample with a substrate (e.g., any of the exemplary substrates described herein) comprising a plurality of capture probes, where a capture probe (e.g., any of the exemplary capture probes described herein) of the plurality comprises a spatial barcode and a capture domain (e.g., any of the exemplary capture domains described herein) that binds specifically to the analyte capture sequence; (c) releasing one or more target analyte(s) from the biological sample, wherein a target analyte of the one or more target analyte(s) that is released from the biological sample is specifically bound by the analyte binding moiety of the analyte capture agent and the analyte capture sequence is specifically bound by the capture domain; (d) extending an end of the capture probe using the analyte capture sequence that is specifically bound by the capture domain of the capture probe as a template, to generate an extended capture probe; (e) exposing the capture probe to: (i) a base (e.g., any of the exemplary bases described herein); and (ii) a detergent (e.g., any of the exemplary detergents described herein), where the exposing results in release of the extended capture probe of step (d) from the substrate; (f) adding a neutralizing agent to the released and extend probe solution; and (g) determining (i) all or a part of a sequence corresponding to the analyte binding moiety barcode or a complement thereof, and (ii) all or a part of a sequence corresponding to the spatial barcode or a complement thereof, and using the determined sequences of (i) and (ii) to determine the location of the target analyte in the biological sample.

In some embodiments, the capture probe can further include a cleavage site (e.g., a cleavage site positioned 5' to the capture domain and the spatial barcode). In some embodiments, the capture probes are attached to the substrate. In some embodiments, the capture probes are attached to a feature, e.g., a bead, a slide, a well, or any of the other exemplary features described herein. In some embodiments, the capture probe can further include a unique molecular identifier (UMI) sequence or a functional domain (e.g., a sequencing handle, a primer binding sequence). In some embodiments, the UMI can be positioned 5' relative to the capture domain.

In some embodiments, the capture domain is positioned at the 3' end of the capture probe. In some embodiments, the target analyte is a nucleic acid. In some embodiments, the nucleic acid is DNA (e.g., genomic DNA). In some embodiments, the nucleic acid is RNA (e.g., mRNA or any of the other types of RNA described herein). In some embodiments where the nucleic acid is an mRNA, the capture domain comprises a poly(T) sequence.

In some embodiments, the target analyte is a protein (e.g., a cell surface protein, an extracellular protein, or an intracellular protein). In some embodiments, the analyte binding moiety comprises an antibody or an antigen-binding fragment thereof.

In some embodiments, the biological sample is a tissue sample. In some embodiments the tissue sample is a fixed tissue sample (e.g., a formalin-fixed paraffin-embedded tissue sample, PFA fixed tissue sample, methanol fixed tissue sample, etc.). In some embodiments, the tissue sample is a fresh tissue sample or a fresh, frozen tissue sample.

In some embodiments, the step of extending an end of the capture probe comprises extending a 3' end of the capture probe using the target analyte that is specifically bound by the capture domain of the capture probe as a template, to generate the extended capture probe. In some embodiments, the extending of the 3' end of the capture probe comprises the use of a reverse transcriptase.

In some embodiments, the step of extending an end of the capture probe comprises extending a 3' end of the capture probe using the analyte capture sequence that is specifically bound by the capture domain of the capture probe as a template, to generate the extended capture probe. In some embodiments, the extending of the 3' end of the capture probe comprises the use of a DNA polymerase (e.g., T7 DNA polymerase, Bsu DNA polymerase, and *E.coli* DNA Polymerase pol I).

In some embodiments, the step of releasing one or more target analyte(s) from the biological sample can include the use of one or more of any of the exemplary permeabilization conditions or agents described herein.

In some embodiments, the detergent can be a nonionic detergent (e.g., Triton-X 100). In some embodiments, the detergent can be an anionic detergent (e.g., sodium dodecyl sulfate (SDS)). In some embodiments, the detergent can be 2-[4-(2,4,4-trimethylpentan-2-yl)phenoxy]ethanol (e.g., Triton X-100^{™}), sodium dodecyl sulphate (e.g., SDS), saponin, polysorbate 80 (e.g., Tween 80^{™}), polysorbate 20 (e.g., Tween 20^{™}), TERGITOL^{™}, or N-lauroylsarcosine sodium salt, or any combination thereof. Additional examples of detergents are known in the art.

In some embodiments, the detergent is present at a concentration of about 0.1% w/v to about 2.0% w/v (e.g., about 0.1% w/v to about 1.8% w/v, about 0.1% w/v to about 1.6% w/v, about 0.1% w/v to about 1.4% w/v, about 0.1% w/v to about 1.2% w/v, about 0.1% w/v to about 1.0% w/v, about 0.1% w/v to about 0.8% w/v, about 0.1% w/v to about 0.6% w/v, about 0.1% w/v to about 0.4% w/v, about 0.1% w/v to about 0.2% w/v, about 0.2% w/v to about 2.0% w/v, about 0.2% w/v to about 1.8% w/v, about 0.2% w/v to about 1.6% w/v, about 0.2% w/v to about 1.4% w/v, about 0.2% w/v to about 1.2% w/v, about 0.2% w/v to about 1.0% w/v, about 0.2% w/v to about 0.8% w/v, about 0.2% w/v to about 0.6% w/v, about 0.2% w/v to about 0.4% w/v, about 0.4% w/v to about 2.0% w/v, about 0.4% w/v to about 1.8% w/v, about 0.4% w/v to about 1.6% w/v, about 0.4% w/v to about 1.4% w/v, about 0.4% w/v to about 1.2% w/v, about 0.4% w/v to about 1.0% w/v, about 0.4% w/v to about 0.8% w/v, about 0.4% w/v to about 0.6% w/v, about 0.6% w/v to about 2.0% w/v, about 0.6% w/v to about 1.8% w/v, about 0.6% w/v to about 1.6% w/v, about 0.6% w/v to about 1.4% w/v, about 0.6% w/v to about 1.2% w/v, about 0.6% w/v to about 1.0% w/v, about 0.6% w/v to about 0.8% w/v, about 0.8% w/v to about 2.0% w/v, about 0.8% w/v to about 1.8% w/v, about 0.8% w/v to about 1.6% w/v, about 0.8% w/v to about 1.4% w/v, about 0.8% w/v to about 1.2% w/v, about 0.8% w/v to about 1.0% w/v, about 1.0% w/v to about 2.0% w/v, about 1.0% w/v to about 1.8% w/v, about 1.0% w/v to about 1.6% w/v, about 1.0% w/v to about 1.4% w/v, about 1.0% w/v to about 1.2% w/v, about 1.2% w/v to about 2.0% w/v, about 1.2% w/v to about 1.8% w/v, about 1.2% w/v to about 1.6% w/v, about 1.2% w/v to about 1.4% w/v, about 1.4% w/v to about 2.0% w/v, about 1.4% w/v to about 1.8% w/v, about 1.4% w/v to about 1.6% w/v, about 1.6% w/v to about 2.0% w/v, about 1.6% w/v to about 1.8% w/v, or about 1.8% w/v to about 2.0% w/v).

In some embodiments, the base can be potassium hydroxide, sodium hydroxide, calcium hydroxide, ammonium hydroxide, magnesium hydroxide, sodium amide, or sodium hydride, or any combination thereof. Additional non-limiting examples of bases are known in the art.

In some embodiments, the base is present at a concentration of about 0.01 M to about 0.5 M (e.g., about 0.01 M to about 0.45 M, about 0.01 M and about 0.40 M, about 0.01 M to about 0.35 M, about 0.01 M to about 0.30 M, about 0.01 M to about 0.25 M, about 0.01 M to about 0.20 M, about 0.01 M to about 0.15 M, about 0.01 M to about 0.10 M, about 0.01 M to about 0.05 M, about 0.01 M to about 0.025 M, about 0.025 M to about 0.5 M, about 0.025 M to about 0.45 M, about 0.025 M and about 0.40 M, about 0.025 M to about 0.35 M, about 0.025 M to about 0.30 M, about 0.025 M to about 0.25 M, about 0.025 M to about 0.20 M, about 0.025 M to about 0.15 M, about 0.025 M to about 0.10 M, about 0.025 M to about 0.05 M, about 0.05 M to about 0.5 M, about 0.05 M to about 0.45 M, about 0.05 M and about 0.40 M, about 0.05 M to about 0.35 M, about 0.05 M to about 0.30 M, about 0.05 M to about 0.25 M, about 0.05 M to about 0.20 M, about 0.05 M to about 0.15 M, about 0.05 M to about 0.10 M, about 0.10 M to about 0.5 M, about 0.10 M to about 0.45 M, about 0.10 M and about 0.40 M, about 0.10 M to about 0.35 M, about 0.10 M to about 0.30 M, about 0.10 M to about 0.25 M, about 0.10 M to about 0.20 M, about 0.10 M to about 0.15 M, about 0.15 M to about 0.5 M, about 0.15 M to about 0.45 M, about 0.15 M and about 0.40 M, about 0.15 M to about 0.35 M, about 0.15 M to about 0.30 M, about 0.15 M to about 0.25 M, about 0.15 M to about 0.20 M, about 0.20 M to about 0.5 M, about 0.20 M to about 0.45 M, about 0.20 M and about 0.40 M, about 0.20 M to about 0.35 M, about 0.20 M to about 0.30 M, about 0.20 M to about 0.25 M, about 0.25 M to about 0.5 M, about 0.25 M to about 0.45 M, about 0.25 M and about 0.40 M, about 0.25 M to about 0.35 M, about 0.25 M to about 0.30 M, about 0.30 M to about 0.5 M, about 0.30 M to about 0.45 M, about 0.30 M and about 0.40 M, about 0.30 M to about 0.35 M, about 0.35 M to about 0.5 M, about 0.35 M to about 0.45 M, about 0.35 M and about 0.40 M, about 0.40 M to about 0.5 M, about 0.40 M to about 0.45 M, or about 0.45 M to about 0.5 M).

In some embodiments, the base is a buffer with a pH of greater than 7 (e.g., about 7.1 to about 14, about 7.1 to about 13.5, about 7.1 to about 13.0, about 7.1 to about 12.5, about 7.1 to about 12.0, about 7.1 to about 11.5, about 7.1 to about 11.0, about 7.1 to about 10.5, about 7.1 to about 10.0, about 7.1 to about 9.5, about 7.1 to about 9.0, about 7.1 to about 8.5, about 7.1 to about 8.0, about 7.1 to about 7.5, about 7.5 to about 14, about 7.5 to about 13.5, about 7.5 to about 13.0, about 7.5 to about 12.5, about 7.5 to about 12.0, about 7.5 to about 11.5, about 7.5 to about 11.0, about 7.5 to about 10.5, about 7.5 to about 10.0, about 7.5 to about 9.5, about 7.5 to about 9.0, about 7.5 to about 8.5, about 7.5 to about 8.0, about 8.0 to about 14, about 8.0 to about 13.5, about 8.0 to about 13.0, about 8.0 to about 12.5, about 8.0 to about 12.0, about 8.0 to about 11.5, about 8.0 to about 11.0, about 8.0 to about 10.5, about 8.0 to about 10.0, about 8.0 to about 9.5, about 8.0 to about 9.0, about 8.0 to about 8.5, about 8.5 to about 14, about 8.5 to about 13.5, about 8.5 to about 13.0, about 8.5 to about 12.5, about 8.5 to about 12.0, about 8.5 to about 11.5, about 8.5 to about 11.0, about 8.5 to about 10.5, about 8.5 to about 10.0, about 8.5 to about 9.5, about 8.5 to about 9.0, about 9.0 to about 14, about 9.0 to about 13.5, about 9.0 to about 13.0, about 9.0 to about 12.5, about 9.0 to about 12.0, about 9.0 to about 11.5, about 9.0 to about 11.0, about 9.0 to about 10.5, about 9.0 to about 10.0, about 9.0 to about 9.5, about 9.5 to about 14, about 9.5 to about 13.5, about 9.5 to about 13.0, about 9.5 to about 12.5, about 9.5 to about 12.0, about 9.5 to about 11.5, about 9.5 to about 11.0, about 9.5 to about 10.5, about 9.5 to about 10.0, about 10.0 to about 14, about 10.0 to about 13.5, about 10.0 to about 13.0, about 10.0 to about 12.5, about 10.0 to about 12.0, about 10.0 to about 11.5, about 10.0 to about 11.0, about 10.0 to about 10.5, about 10.5 to about 14, about 10.5 to about 13.5, about 10.5 to about 13.0, about 10.5 to about 12.5, about 10.5 to about 12.0, about 10.5 to about 11.5, about 10.5 to about 11.0, about 11.0 to about 14, about 11.0 to about 13.5, about 11.0 to about 13.0, about 11.0 to about 12.5, about 11.0 to about 12.0, about 11.0 to about 11.5, about 11.5 to about 14, about 11.5 to about 13.5, about 11.5 to about 13.0, about 11.5 to about 12.5, about 11.5 to about 12.0, about 12.0 to about 14, about 12.0 to about 13.5, about 12.0 to about 13.0, about 12.0 to about 12.5, about 12.5 to about 14, about 12.5 to about 13.5, about 12.5 to about 13.0, about 13.0 to about 14, about 13.0 to about 13.5, or about 13.5 to about 14).

In some embodiments, the exposing the extended capture probe to the base and the detergent can be performed at a temperature of about 30 °C to about 80 °C (e.g., about 30 °C to about 75 °C, about 30 °C to about 70 °C, about 30 °C to about 65 °C, about 30 °C to about 60 °C, about 30 °C to about 55 °C, about 30 °C to about 50 °C, about 30 °C to about 45 °C, about 30 °C to about 40 °C, about 30 °C to about 35 °C, about 35 °C to about 80 °C, about 35 °C to about 75 °C, about 35 °C to about 70 °C, about 35 °C to about 65 °C, about 35 °C to about 60 °C, about 35 °C to about 55 °C, about 35 °C to about 50 °C, about 35 °C to about 45 °C, about 35 °C to about 40 °C, about 40 °C to about 80 °C, about 40 °C to about 75 °C, about 40 °C to about 70 °C, about 40 °C to about 65 °C, about 40 °C to about 60 °C, about 40 °C to about 55 °C, about 40 °C to about 50 °C, about 40 °C to about 45 °C, about 45 °C to about 80 °C, about 45 °C to about 75 °C, about 45 °C to about 70 °C, about 45 °C to about 65 °C, about 45 °C to about 60 °C, about 45 °C to about 55 °C, about 45 °C to about 50 °C, about 50 °C to about 80 °C, about 50 °C to about 75 °C, about 50 °C to about 70 °C, about 50 °C to about 65 °C, about 50 °C to about 60 °C, about 50 °C to about 55 °C, about 55 °C to about 80 °C, about 55 °C to about 75 °C, about 55 °C to about 70 °C, about 55 °C to about 65 °C, about 55 °C to about 60 °C, about 60 °C to about 80 °C, about 60 °C to about 75 °C, about 60 °C to about 70 °C, about 60 °C to about 65 °C, about 65 °C to about 80 °C, about 65 °C to about 75 °C, about 65 °C to about 70 °C, about 70 °C to about 80 °C, about 70 °C to about 75 °C, or about 75 °C to about 80 °C).

In some embodiments, the step of exposing is performed for, e.g., about 1 minute to about 6 hours (e.g., about 1 minute to about 5 hours, about 1 minute to about 4 hours, about 1 minute to about 3 hours, about 1 minute to about 2 hours, about 1 minutes to about 1 hours, about 1 minute to about 50 minutes, about 1 minute to about 40 minutes, about 1 minute to about 30 minutes, about 1 minute to about 25 minutes, about 1 minute to about 20 minutes, about 1 minute to about 15 minutes, about 1 minute to about 10 minutes, about 1 minute to about 5 minutes, about 5 minutes to about 6 hours, about 10 minutes to about 6 hours, about 10 minutes to about 6 hours, about 15 minutes to about 6 hours, about 20 minutes to about 6 hours, about 25 minutes to about 6 hours, about 30 minutes to about 6 hours, about 40 minutes to about 6 hours, about 50 minutes to about 6 hours, about 1 hour to about 6 hours, about 2 hours to about 6 hours, about 3 hours to about 6 hours, about 4 hours to about 6 hours, or about 5 hours to about 6 hours).

Some embodiments of the methods further include, following the release of the extended capture probe from the substrate, transferring the released extended capture probe into a receptacle, and adding a neutralizing agent to the receptacle including the released extended capture probes. In some embodiments, the neutralizing agent can include, but is not limited to, an acid, such as Tris-hydrochloric acid, sulfonic acid, sulfuric acid, hydrochloric acid, or any other acids described herein or known in the art. In some embodiments, the neutralizing agent can be a buffer. In some embodiments, the buffer can include a weak acid or base.

In some embodiments, the neutralization buffer can be, but is not limited to, 2-amino-2-(hydroxymethyl)propane-1,3-diol, 2-[4-(2-hydroxyethyl)piperazin-1-yl]ethanesulfonic acid, 4-(2-hydroxyethyl)-1-piperazineethanesulfonic acid (HEPES), 3-morpholinopropane-1-sulfonic acid (MOPS), piperazine-N,N'-bis(2-ethanesulfonic acid) (PIPES), 2-(N-morpholino)ethanesulfonic acid (MES), 2-(Bis(2-hydroxyethyl)amino)acetic acid (Bicine), N-(2-Hydroxy-1,1-bis(hydroxymethyl)ethyl)glycine (Tricine), 3-{[1,3-dihydroxy-2-(hydroxymethyl)propan-2-yl]amino}propane-1-sulfonic acid (TAPS), 3-[[1,3-dihydroxy-2-(hydroxymethyl)propan-2-yl]amino]-2-hydroxypropane-1-sulfonic acid (TAPSO), tris(hydroxymethyl)aminomethane (Tris), sodium carbonate, Tris hydrochloride, potassium phosphate, or disodium hydrogen phosphate, or any combination thereof.

In some embodiments, the neutralizing agent can be added to bring the pH of a solution comprising the released extended capture probe to a pH of about 6.0 to about 8.0 (e.g., about 6.0 to about 7.8, about 6.0 to about 7.6, about 6.0 to about 7.4, about 6.0 to about 7.2, about 6.0 to about 7.0, about 6.0 to about 6.8, about 6.0 to about 6.6, about 6.0 to about 6.4, about 6.0 to about 6.2, about 6.2 to about 8.0, about 6.2 to about 7.8, about 6.2 to about 7.6, about 6.2 to about 7.4, about 6.2 to about 7.2, about 6.2 to about 7.0, about 6.2 to about 6.8, about 6.2 to about 6.6, about 6.2 to about 6.4, about 6.4 to about 8.0, about 6.4 to about 7.8, about 6.4 to about 7.6, about 6.4 to about 7.4, about 6.4 to about 7.2, about 6.4 to about 7.0, about 6.4 to about 6.8, about 6.4 to about 6.6, about 6.6 to about 8.0, about 6.6 to about 7.8, about 6.6 to about 7.6, about 6.6 to about 7.4, about 6.6 to about 7.2, about 6.6 to about 7.0, about 6.6 to about 6.8, about 6.8 to about 8.0, about 6.8 to about 7.8, about 6.8 to about 7.6, about 6.8 to about 7.4, about 6.8 to about 7.2, about 6.8 to about 7.0, about 7.0 to about 8.0, about 7.0 to about 7.8, about 7.0 to about 7.6, about 7.0 to about 7.4, about 7.0 to about 7.2, about 7.2 to about 8.0, about 7.2 to about 7.8, about 7.2 to about 7.6, about 7.2 to about 7.4, about 7.4 to about 8.0, about 7.4 to about 7.8, about 7.4 to about 7.6, about 7.6 to about 8.0, about 7.6 to about 7.8, or about 7.8 to about 8.0).

Some embodiments of the methods described herein further include determining an amount of the extended capture probe released from the substrate. In some embodiments, the amount of extended capture probe released from the substrate can be determined using, e.g., nucleic acid amplification. In some embodiments, the amount of extended capture probe released from the substrate can be determined using optical methods, e.g., hybridization of a fluorophore-conjugated probe.

Some embodiments of any of the methods described herein can further include comparing the amount of extended capture probe released from the substrate to a reference level. The reference level can be, e.g., produced by a control method that can include the performance of the steps described herein but can use one or more different parameter or one or more different steps. For example, the different parameter can include a different biological sample, a different set of reagents, a different condition, or any combination thereof.

In some embodiments, the step of determining comprising sequencing (i) all or a part of the sequence corresponding to the target analyte specifically bound by the capture domain or the complement thereof, and (ii) all or a part of the sequence corresponding to the spatial barcode or the complement thereof. In some embodiments, the step of determining comprising sequencing (i) all or a part of the sequence corresponding to the analyte binding moiety barcode or the complement thereof, and (ii) all or part of the sequence corresponding to the spatial barcode or the complement thereof. In some embodiments, the sequencing is high throughput next generation sequencing (e.g., Illuminia sequencing).

### Kits

A kit comprising a substrate comprising a base (e.g., any of the exemplary bases described herein), a detergent (e.g., any of the exemplary detergents described herein), and a substrate comprising a plurality of capture probes (e.g., any of the exemplary capture probes described herein), where a capture probe of the plurality comprises a capture domain. In some examples, the kits can further include a reverse transcriptase. In some examples, the detergent is a non-ionic detergent (e.g., Triton-X 100). In some examples, the detergent is an anionic detergent (e.g., sodium dodecyl sulfate (SDS)). In some examples, the detergent is present at a concentration of about 0.1% w/v to about 2.0% w/v (or any of the subranges of this range described herein). In some examples, the base is potassium hyrdroxide. In some examples, the base is present at a concentration of about 0.01 M to about 0.3 M (or any of the subranges of this range described herein). In some examples, the kit further includes a neutralizing agent (e.g., an acid or a buffer, e.g., 2-amino-2-(hydroxymethyl)propane-1,3-diol or 2-[4-(2-hydroxyethyl)piperazin-1-yl]ethanesulfonic acid). In some examples, the kit further includes instructions for performing any of the methods described herein.

Also provided are kits that include: a plurality of analyte capture agents (e.g., any of the exemplary analyte capture agents described herein), where an analyte capture agent of the plurality of analyte capture agents comprises an analyte binding moiety, analyte binding moiety barcode, and an analyte capture sequence; a substrate comprising a plurality of capture probes (e.g., any of the exemplary capture probes described herein), wherein a capture probe of the plurality comprises a capture domain that binds specifically to the analyte capture sequence; a base; and a detergent. In some examples, the kit further includes a DNA polymerase. In some examples, the detergent is a non-ionic detergent (e.g., Triton-X 100). In some examples, the detergent is an anionic detergent (e.g., SDS). In some examples, the detergent is present at a concentration of about 0.1% w/v to about 2.0% w/v (e.g., or any of the subranges of this range described herein). In some examples, the base is potassium hydroxide. In some examples, the base is present at a concentration of about 0.01 M to about 0.3 M (e.g., or any of the subranges of this range described herein). In some examples, the kit further includes a neutralizing agent (e.g., an acid or a buffer, e.g., 2-amino-2-(hydroxymethyl)propane-1,3-diol or 2-[4-(2-hydroxyethyl)piperazin-1-yl]ethanesulfonic acid). In some examples, the analyte binding moiety comprises an antibody or an antigen-binding fragment thereof. In some examples, the kit further includes instructions for performing any of the methods described herein.

### Exemplary Methods

Some embodiments of the methods described herein can include fixing the biological sample by removing a slide with tissue from storage (e.g., -80 °C) and heating the slide for 1 minute at 37 °C (e.g., by placing slides on a thermocycler adapter ina pre-heated thermocycler). Immediately after heating, the back of the slide is dried with a Kimwipe, and the slide is placed into a slide mailer, which is filled with chilled 100% methanol. The slide is incubated in the chilled 100% methanol for 30 minutes at -20 °C. The slide is dried (e.g., removing any residual methanol using a Kimwipe), and the slide is placed on a flat clean surface.

Some embodiments of the methods described herein further include fixing and staining the biological sample disposed on a slide. For example, isopropanol can be pipetted onto the slide and allowed to incubate for 1 minute. The isopropanol is removed from the slide (e.g., removing any residual isopropanol using a Kimwipe). Once the slide is completely dry, hematoxylin is pipetted onto the slide and incubated for 7 minutes. The slide is dipped in ultrapure water five times and into a first beaker of ultrapure water fifteen times. The slide is dipped into a second beaker of ultrapure water fifteen times. The water is removed from the slide (e.g., any residual water being removed using a Kimwipe). Blueing buffer is pipetted onto the slide and the slide incubated for 2 minutes at room temperature. The slide is dipped in the second beaker of of ultrapure water five times. Eosin mix is pipetted onto the slide and the slide incubated for 1 minute at room temperature and dipped into a third beaker of ultrapure water ten to fifteen times. The slide is air-dried and incubated for 5 minutes at 37 °C by placing the slide on a Thermocycler Adapter in a pre-heated thermocycler. The slide can then be imaged before performing permeabilization of the biological sample.

Permeabilization of the slide can be performed by placing the slide into a slide cassette (e.g., any of the exemplary slide casettes described herein). A pre-heated permeabilization enzyme (e.g., pepsin) is added to the slide and the slide cassette sealed. The slide is incubated at 37 °C, and after this incubation, the permeabilization enzyme is removed from the slide. The slide is washed by slowing adding 0.1X SSC to cover the slide.

Extension of a capture probe can be performed by adding 1 µM RT reagent, 20 µM reducing agent, and optionally, approximately 70 µM template switching oligo. The extension reaction is performed under the following conditions: pre-heating at 53 °C, reverse transcription at 53 °C for 45 minutes, and holding at 4 °C.

For removal of the extended capture probes from the slide, the slides are contacted with 0.08 M KOH and 1% Trion-X in Nuclease-Free Water. The removal is performed using the following thermocycle settings: pre-heating at 65 °C, 65 °C for 15 minutes, and a holding step at 4 °C. After thermocycling, the released extended capture probes (70 µL) are placed in a new tube and neutralized with 10 µL of 1 M Tris, pH 7.0.

In some examples, the released extended capture probes are amplified using a solution comprising primers and an amplification mix, and using a thermocycler: denaturation at 98 °C for 3 minutes, denaturation for 15 seconds at 98 °C, annealing for 20 seconds at 63 °C, extension for 1 minute at 72 °C, and a holding step at 4 °C.

### EXAMPLES

### Example 1. Release of extended capture probes from a substrate

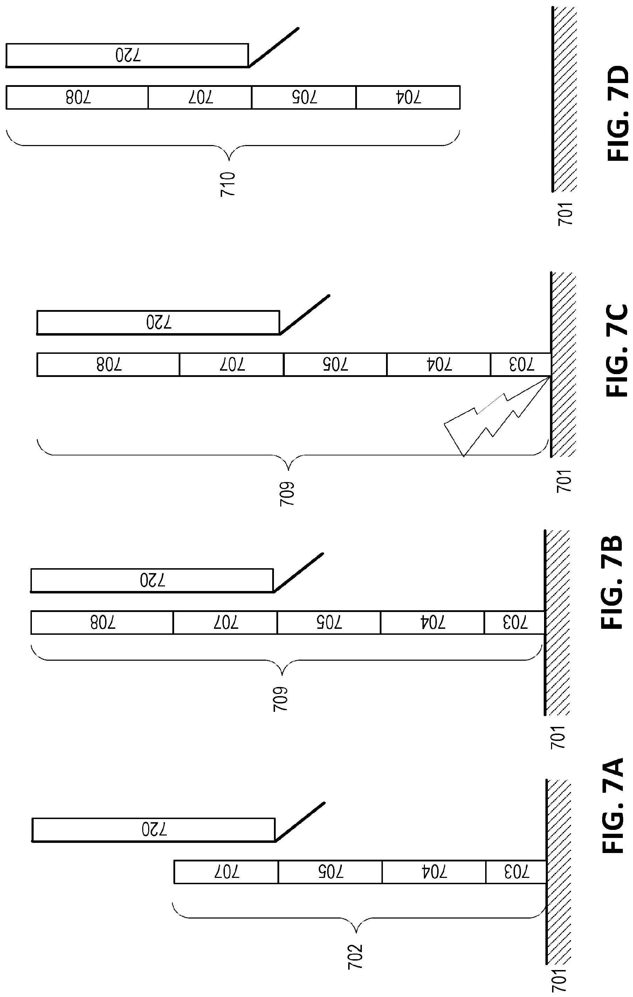

An exemplary workflow for the release of an extended capture probe is shown in **FIG. 7A** through **FIG. 7D. FIG. 7A** depicts a capture probe **702** comprising a cleavage domain **703,** functional sequence **704,** spatial barcode **705,** and a capture domain **707** immobilized on a substrate **701.** The functional sequence **704** can be any of the exemplary functional sequences described herein. The spatial barcode **705** can be any spatial barcode sequence as described herein. The capture domain **707** can include a sequence that specifically hybridizes to a target analyte. The target analyte **720** shown in **FIG. 7A** is depicted as an mRNA target analyte, but the analyte **720** can be any target analyte or analyte capture sequence as described herein.

As shown in **FIG. 7B****,** after the target analyte **720** is bound to the capture domain **707** of the capture probe **702,** a reverse transcriptase can is used to extend the 3' end of the capture probe, creating a first strand complement **708** to a sequence present in the target analyte **720,** to generate an extended capture probe **709.**

**FIG. 7C** shows that the extended capture probe **709** can be exposed to a base and a detergent (lighting bolt icon) to release the extended capture probe **709** from the substrate at the cleavage domain **703.** Optionally, exposing the extended capture probe **709** to the base and detergent can include raising the temperature of the base and the detergent.

**FIG. 7D** shows the released capture probe **710** after its release from the substrate. **FIG. 7D** further depicts the optional removal of the cleavage domain **703** of the extended capture probe **709.** Following the release of the extended capture probe **710** from the substrate, the solution containing the released and extended capture probe **710** can be transferred to a fresh container and optionally neutralized before being used to generate a library for analyte capture determinations (e.g., using any of the exemplary methods described herein).

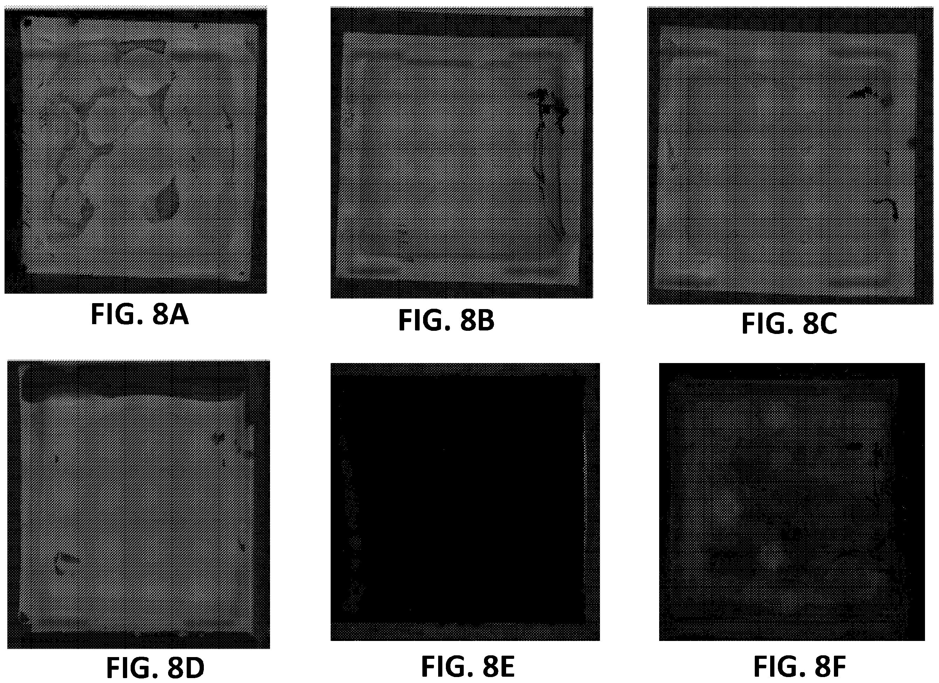

**FIGS. 8A-F** demonstrate different conditions comparative to a control that were evaluated for their ability to release capture probes from a substrate. Briefly, the slides with immobilized capture probes were subjected to different conditions (the control slide was not treated for release of capture probes) and incubated at a specific temperature for 10 min., residual solutions were removed and any remaining capture probes were labeled with Cy3 and fluorescently detected. **FIG. 8A****,** the control slide, shows a substrate with attached capture probes demonstrating a high degree of fluorescence. **FIG. 8B** shows a substrate with attached capture probes following exposure to 0.1 N KOH and 1 % w/v SDS at room temperature. **FIG. 8C** shows a substrate with attached capture probes following exposure to 0.1 N KOH at room temperature. **FIG. 8D** shows a substrate with attached capture probes following exposure to 1 % w/v SDS and proteinase K (2.5mg/ml) at 50 °C. **FIG. 8E** shows a substrate with attached capture probes following exposure to 0.1 N KOH and 1 % w/v SDS at 50 °C. Removal of the attached capture probes is demonstrated by the lack of fluorescence in the image compared to the control image of **FIG. 8A. FIG. 8F** shows a substrate with attached capture probes following exposure to 0.1 N KOH at 50 °C. These data indicate that the combination of base and detergent, and optionally, heat, result in release of capture probes from an array as evidenced by the minimal to undetectable levels of fluorescence seen in **FIGs. 8E** and **8F****.**

A further set of experiments was performed to determine the location of mRNAs in a mouse brain section using one of two different workflows: a control workflow and an exemplary method described herein (test workflow) ("KOH-triton strip"). The control workflow is the spatial workflow described herein which generates a second strand complementary to the extended capture probe using a template-switching oligonucleotide and the generation of a library using the same. The test workflow, instead of generating a second strand, includes releasing the extended capture probe described herein, by incubation with potassium hydroxide and Triton X-100^{™} for 15 minutes at 65 °C, removing the released extended capture probes from the slide and neutralizing the released extended capture probe containing solution with 1M Tris, pH 7.0, followed by standard cDNA amplification and library construction.

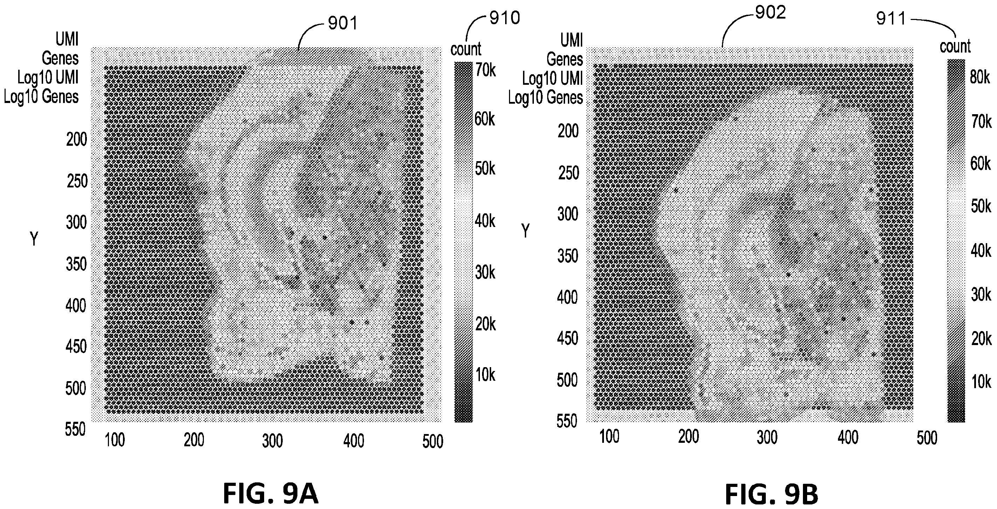

To perform a comparison of the two testing methods, four mouse brain sections were disposed on four spatially-barcoded arrays and standard cDNA amplification and library generation methods performed (two sections processed using the control workflow and two sections processed using the test workflow). **FIG. 9A** shows an exemplary gene expression heat map **901** of a mouse brain section affixed to a spatially barcoded array generated using the control workflow. The scale bar **910** on the right of the gene expression heat map **901** shows the heat map color scale in UMI count from 0 (e.g., blue) to 70,000 (e.g., red).

**FIG. 9B** shows an exemplary gene expression heat map **902** of a mouse brain section affixed to a spatially-barcoded array generated using the test workflow. The scale bar **911** on the right of the gene expression heat map **902** shows the heat map color scale in UMI count from 0 (e.g., blue) to about 85,000 (e.g., red).

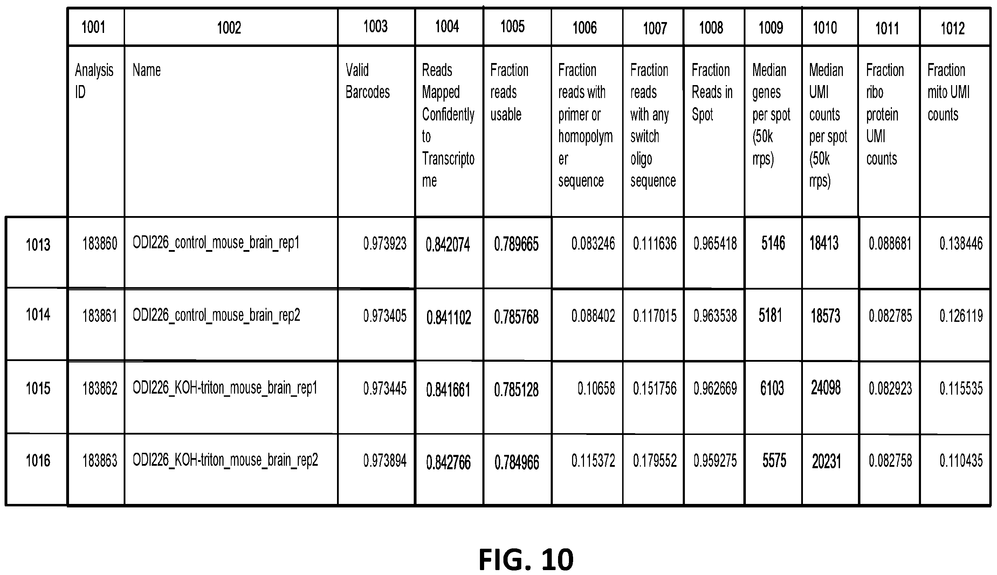

The properties of the two libraries generated using the control workflow **(1013** and **1014)** and the two libraries generated using the test workflow (KOH-Triton) **(1015** and **1016)** is shown in **FIG. 10****.** Library **1013** corresponds to the control heat map **901** of mouse brain image in **FIG. 9A** and library **1015** corresponds to test heat map **902** of mouse brain image in **FIG. 9B****.** The control workflow and the test workflow show comparable sequencing metrics. The data indicate that the methods provided herein provide for an accurate and more efficient option for generating a library as compared to the control.

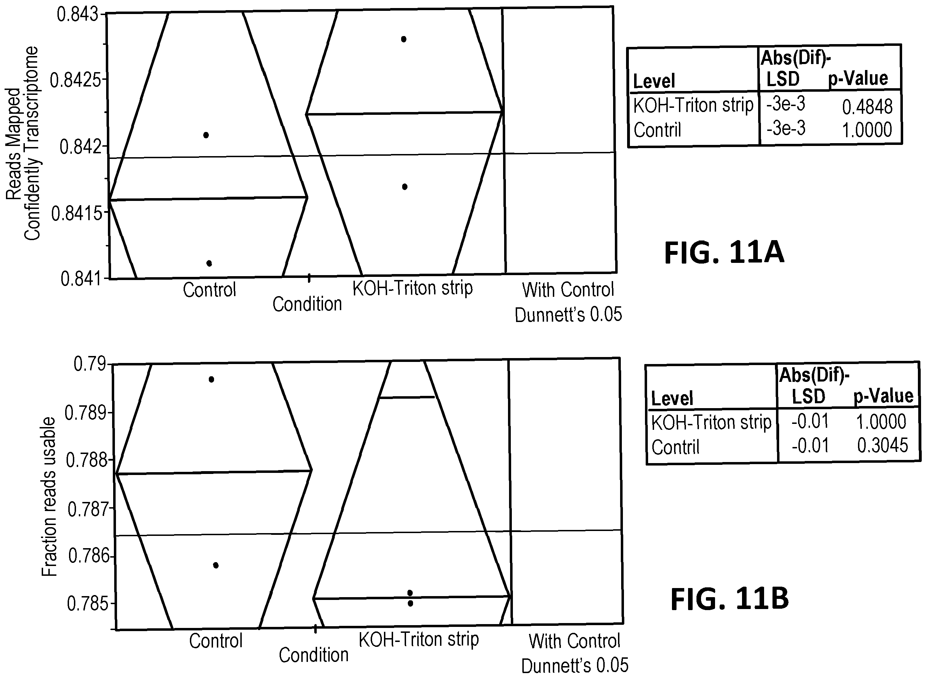

Select specific properties of the two libraries generated using the control workflow and the two libraries generated using the test workflow (KOH-Triton) are shown in **FIGS. 11A-11F****.** The data were compared using Dunnett's test for each figure. In general, the left grouping of data in **FIGS. 11A-11F** show data from the libraries generated using the control workflow and the right grouping of data in **FIGS. 11A-11F** show data from the libraries generated using the test method. **FIGS. 11A-11F** further depict the p-value comparison between the data from libraries generated using the control workflow and the data from libraries generated using the test workflow.

**FIG. 11A** shows a chart depicting the fraction of reads mapped confidently to the biological sample transcriptome, e.g., column **1004** of **FIG. 10****.** The scale of the y-axis depicted is from 0.841 to 0.843. **FIG. 11B** shows a chart depicting the fraction of usable reads, e.g., column **1005** of **FIG. 29.** The scale of the y-axis depicted is from about 0.785 to 0.79. The data in **FIG. 11A** and **FIG. 11B** indicate that there is no significant difference in transcriptome mapping confidence or usable read count between the libraries generated using the control workflow and libraries generated using the test workflow.

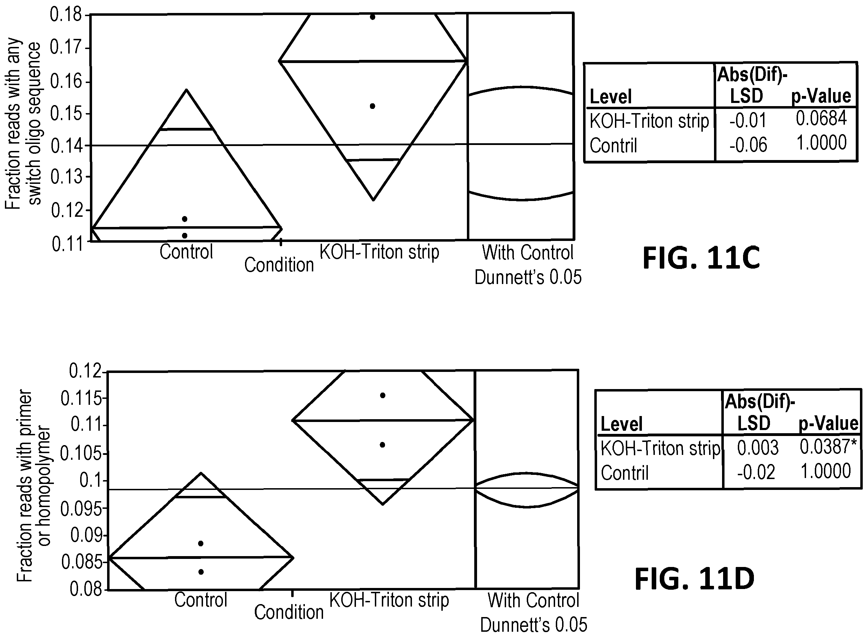

**FIG. 11C** shows a chart depicting the fraction of usable reads with any TSO sequence, e.g., column **1007** of **FIG. 10****.** The scale of the y-axis depicted is from 0.11 to 0.18. **FIG. 11D** shows a chart depicting the fraction of usable reads with a primer or homopolymer sequence, e.g., column **1006** of **FIG. 10****.** The scale of the y-axis depicted is from 0.08 to 0.12. **FIG. 11D** shows a slightly higher primer or homopolymer sequence contamination in the libraries generated using the test workflow, e.g., rows **1015** and **1016** of **FIG. 10****.** This is likely due to all barcodes being released during the test workflow as opposed to the second strand generated in the control workflow being the only barcodes being carried through to amplification.

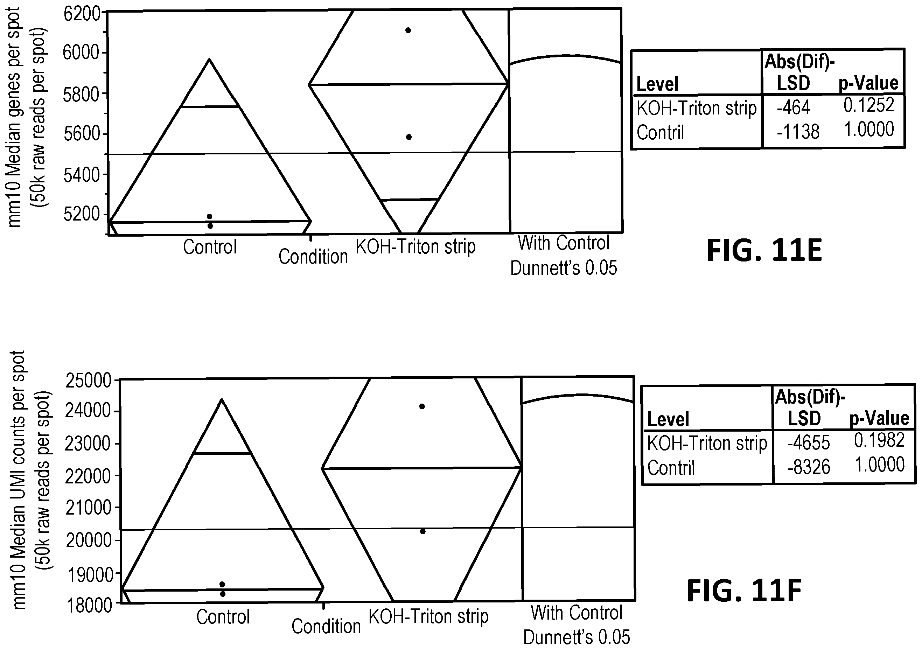

**FIG. 11E** shows a chart depicting the median genes read per spot on the spatially barcoded arrays, e.g., column **1009** of **FIG. 10****.** The scale of the y-axis depicted is from about 5100 genes to about 6200 genes. **FIG. 11F** shows a chart depicting the median UMI count per spot, e.g., column **1010** of **FIG. 10****.** **FIG. 11E** and **FIG. 11F** show slightly increased median gene and UMI count for libraries generated using the test method when compared to libraries generated using the control method, although the difference is not significant (<0.005).

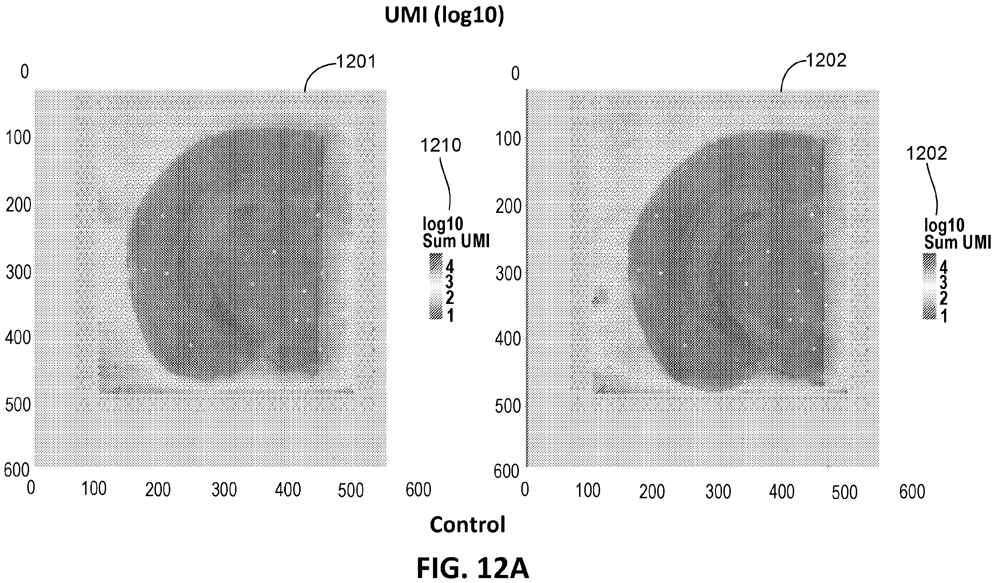

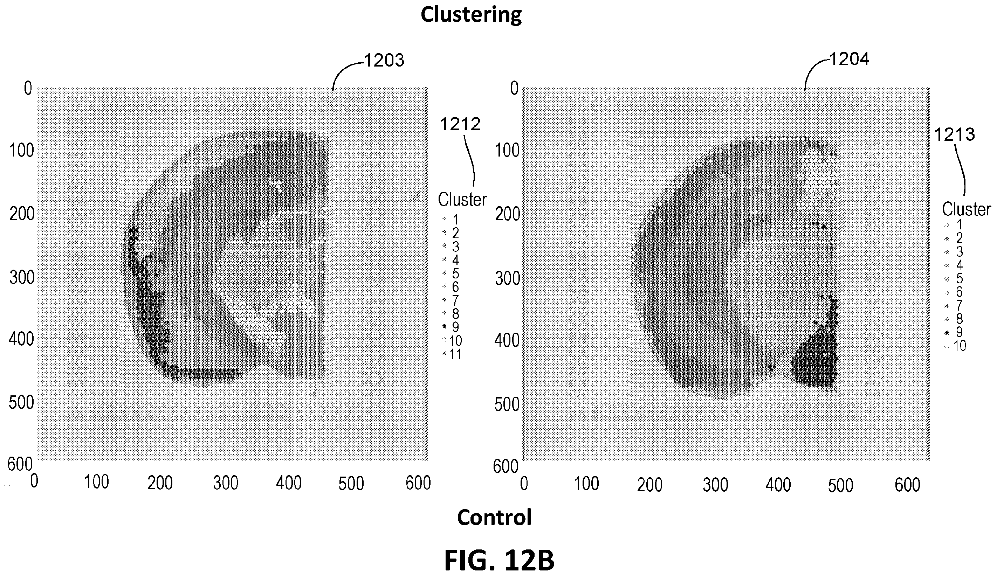

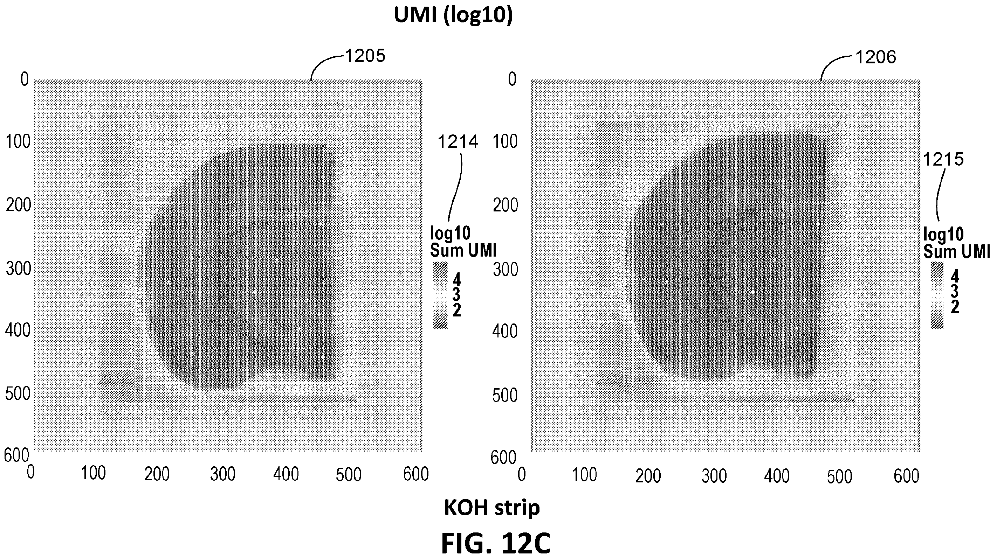

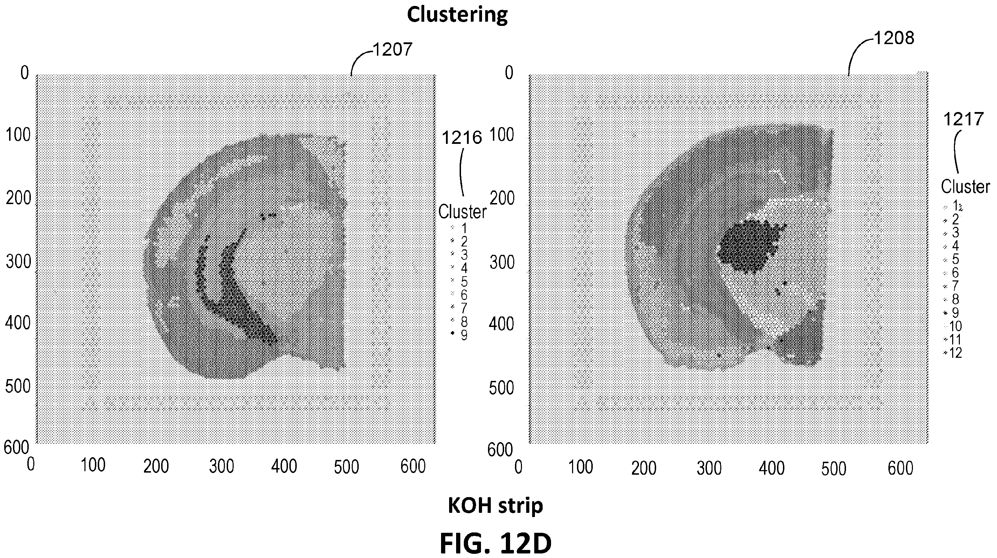

Performing a further comparison of the two testing methods, the process of **FIGs. 9A** and **9B** was repeated for four mouse brain section replicates. The sections were disposed on four spatially-barcoded arrays and standard cDNA amplification and library generation methods were performed (two sections processed using the control workflow and two sections processed using the test workflow). **FIGS. 12A** and **12C** show gene expression heat maps and **FIGS. 12B** and **12D** show gene clustering maps from the generated libraries. **FIG. 12A** shows two gene expression heat maps, **1201** and **1202,** of two exemplary mouse brain sections affixed to a spatially barcoded array generated using the control workflow. The scale bar **1210** on the right of the gene expression heat map **1201** shows the heat map color scale in log10 UMI count from 0 (e.g., blue) to 5 (e.g., red). The scale bar **1211** on the right of the gene expression heat map **1202** shows the heat map color scale in log10 UMI count from 0 (e.g., blue) to about 5 (e.g., red). **FIG. 12B** shows two gene expression clustering maps, **1203** and **1204,** of two mouse brain sections affixed to a spatially barcoded array generated using the control workflow. The scale bar **1212** on the right of the gene expression clustering map **1203** shows the clustering map clustering ID from 1 to 11. The scale bar **1213** on the right of the gene expression clustering map **1203** shows the clustering map clustering ID from 1 to 10. Each clustering ID corresponds to the primary genes detected at the spatial location.

**FIG. 12C** shows two gene expression heat maps, **1205** and **1206,** of two exemplary mouse brain sections affixed to a spatially barcoded array generated using the test workflow (KOH-Triton). The scale bar **1214** on the right of the gene expression heat map **1205** shows the heat map color scale in log10 UMI count from 0 (e.g., blue) to 5 (e.g., red). The scale bar **1215** on the right of the gene expression heat map **1206** shows the heat map color scale in log10 UMI count from 0 (e.g., blue) to about 5 (e.g., red).

**FIG. 12D** shows two gene expression clustering maps, **1207** and **1208,** of two mouse brain sections affixed to a spatially barcoded array generated using the test workflow (KOH-Triton). The scale bar **1216** on the right of the gene expression clustering map **1207** shows the clustering map clustering ID from 1 to 9. The scale bar **1217** on the right of the gene expression clustering map **1208** shows the clustering map clustering ID from 1 to 12. Each clustering ID corresponds to the primary genes detected at the spatial location.

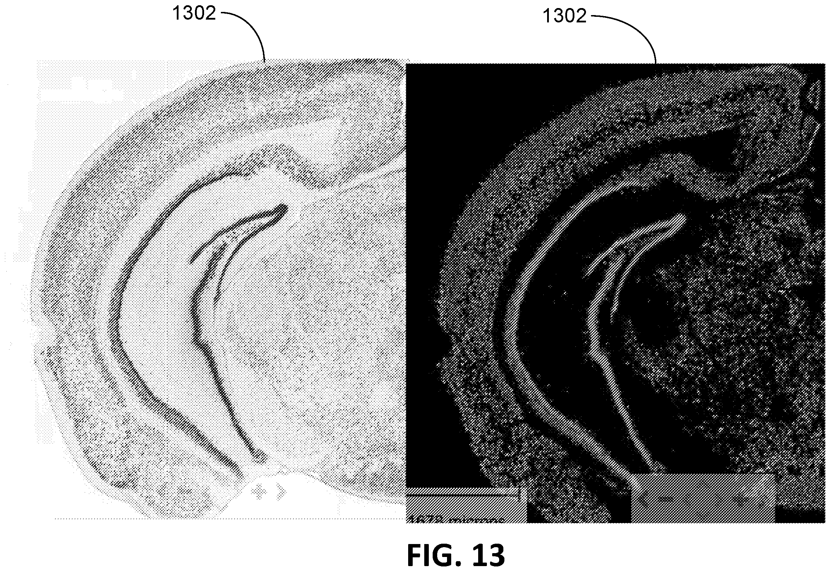

**FIG. 13** shows two *in situ* hybridization (ISH) image identifying hippocalcin gene expression in bright-field, left, and fluorescence, right.

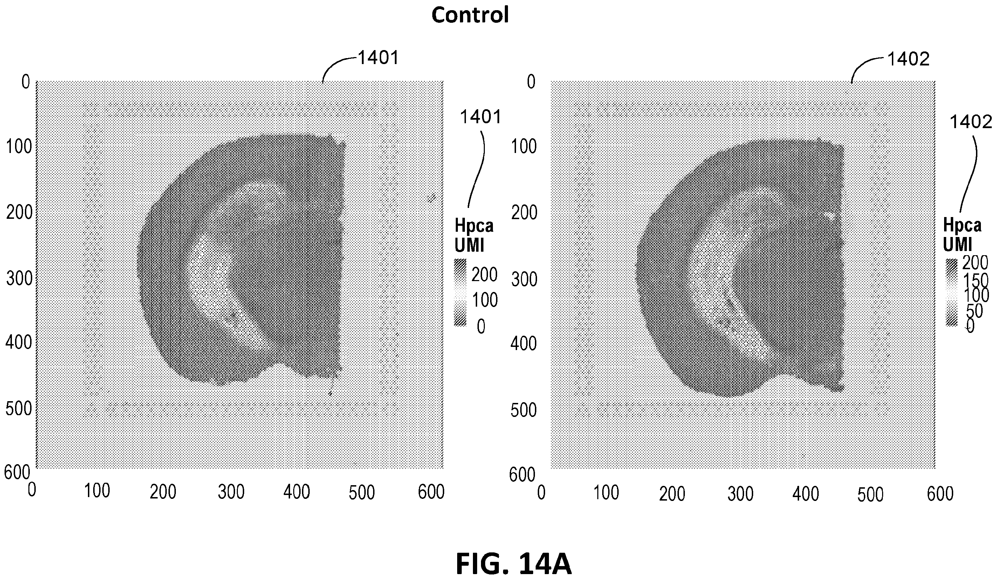

**FIG. 14A** shows two hippocalcin gene expression heat maps, **1401** and **1402,** of mouse brain sections affixed to a spatially barcoded array generated using the control workflow. The scale bar **1410** on the right of the gene expression heat map **1401** shows the heat map color scale in hippocalcin-related UMI count from 0 (e.g., blue) to 250 (e.g., red). The scale bar **1411** on the right of the gene expression heat map **1402** shows the heat map color scale in hippocalcin-related UMI count from 0 (e.g., blue) to about 200 (e.g., red).

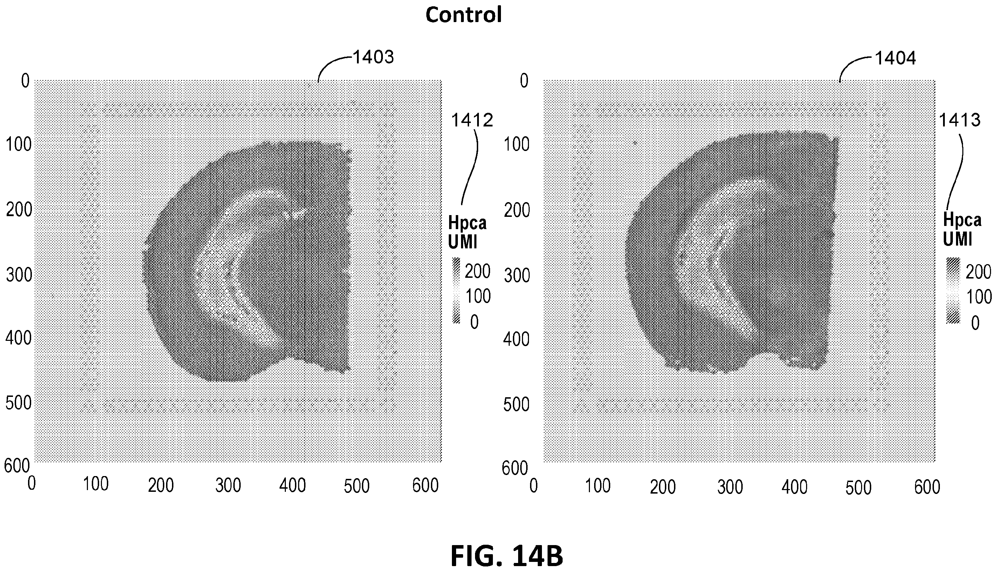

**FIG. 14B** shows two hippocalcin gene expression heat maps, **1403** and **1404,** of mouse brain sections affixed to a spatially barcoded array generated using the test workflow. The scale bar **1412** on the right of the gene expression heat map **1403** shows the heat map color scale in hippocalcin-related UMI count from 0 (e.g., blue) to 250 (e.g., red). The scale bar **1413** on the right of the gene expression heat map **1404** shows the heat map color scale in hippocalcin-related UMI count from 0 (e.g., blue) to about 200 (e.g., red). The *in situ* hybridization images of hippocalcin gene expression **(****FIG. 13****)** correlate with the gene expression heat maps from both the control workflow (e.g., second strand synthesis and collection) **(****FIG. 14A****)** and the test workflow (e.g., KOH strip) **(****FIG. 14B****).**

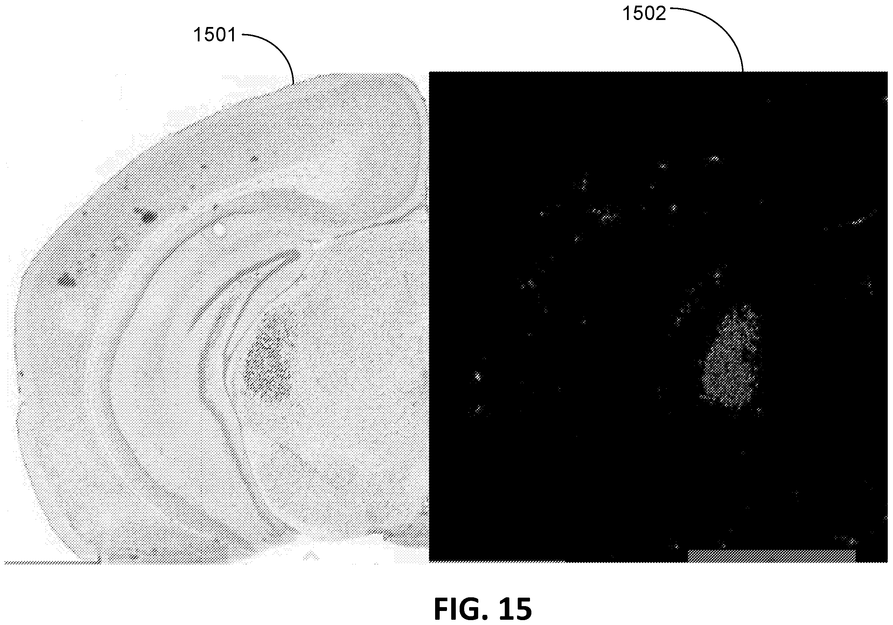

**FIG. 15** shows two in situ hybridization (ISH) images identifying protein kinase c alpha gene expression in bright-field, left, and fluorescence, right.

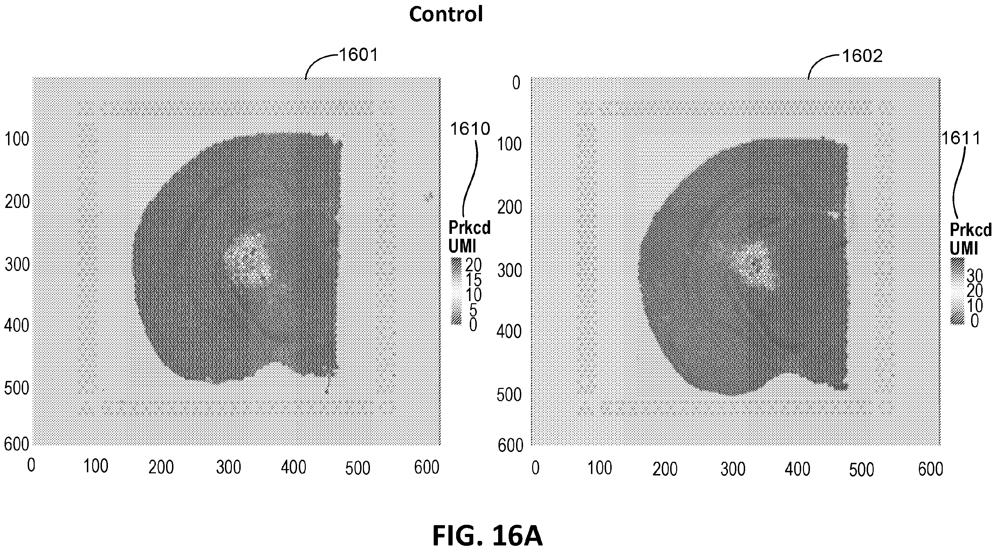

**FIG. 16A** shows two protein kinase c alpha gene expression heat maps, **1601** and **1602,** of mouse brain sections affixed to a spatially barcoded array generated using the control workflow. The scale bar **1610** on the right of the gene expression heat map **1601** shows the heat map color scale in protein kinase c alpha-related UMI count from 0 (e.g., blue) to 20 (e.g., red). The scale bar **1611** on the right of the gene expression heat map **1602** shows the heat map color scale in protein kinase c alpha-related UMI count from 0 (e.g., blue) to about 35 (e.g., red).

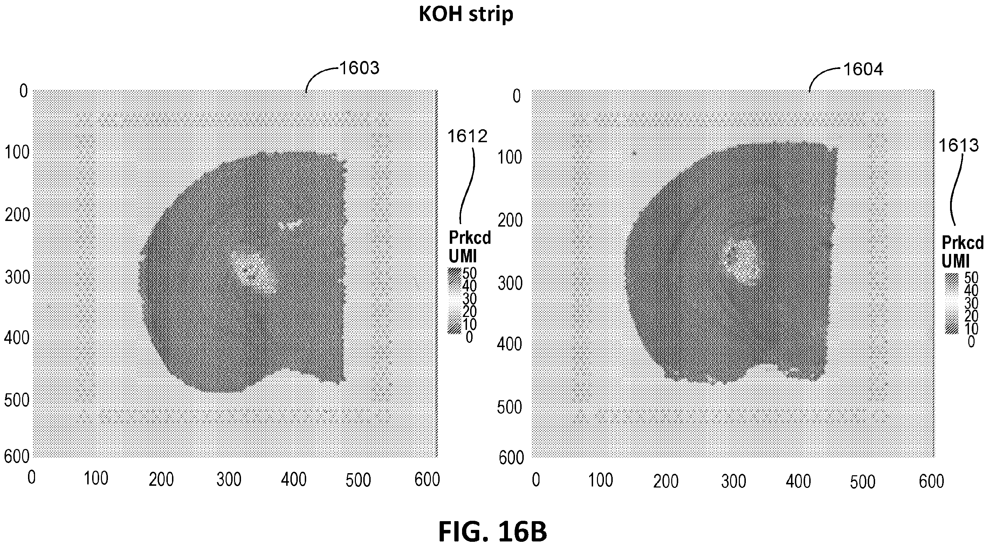

**FIG. 16B** shows two protein kinase c alpha gene expression heat maps, **1603** and **1604,** of mouse brain sections affixed to a spatially barcoded array generated using the test workflow. The scale bar **1612** on the right of the gene expression heat map **1603** shows the heat map color scale in protein kinase c alpha-related UMI count from 0 (e.g., blue) to 50 (e.g., red). The scale bar **1613** on the right of the gene expression heat map **1604** shows the heat map color scale in protein kinase c alpha-related UMI count from 0 (e.g., blue) to about 50 (e.g., red).

The *in situ* hybridization images of hippocalcin gene expression **(****FIG. 15****)** correlate with the gene expression heat maps from both the control workflow **(****FIG. 16A****)** and the test workflow (e.g., KOH strip) **(****FIG. 16B****).**

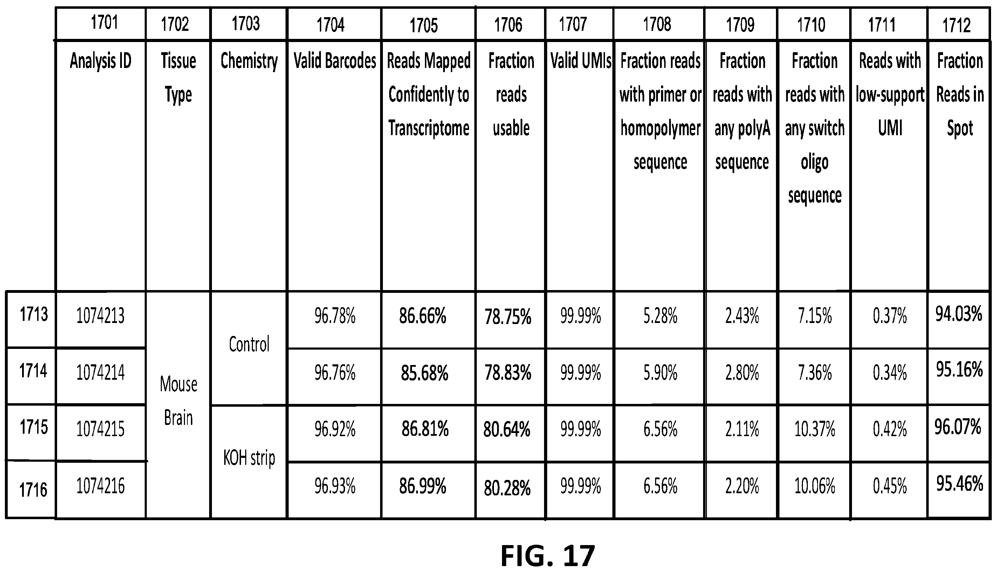

The properties of the two libraries generated using the control workflow **(1713** and **1714)** and the two libraries generated using the test workflow (KOH-Triton) **(1715** and **1716)** is shown in **FIG. 17****.** Library **1713** corresponds to control heat maps **1401** and **1601** and library **1714** corresponds to control heat maps **1402** and **1602** of mouse brain images in **FIGS. 14A** and **16A****.** Library **1715** corresponds to test heat map **1403** and **1603** and library **1716** corresponds to test heat map **1404** and **1604** of mouse brain image in **FIGS. 14B** and **16B**. The control workflow and the test workflow show comparable sequencing metrics, and libraries **1715** and **1716** show increased fraction usable reads **1706** and fraction reads in spot **1712.**

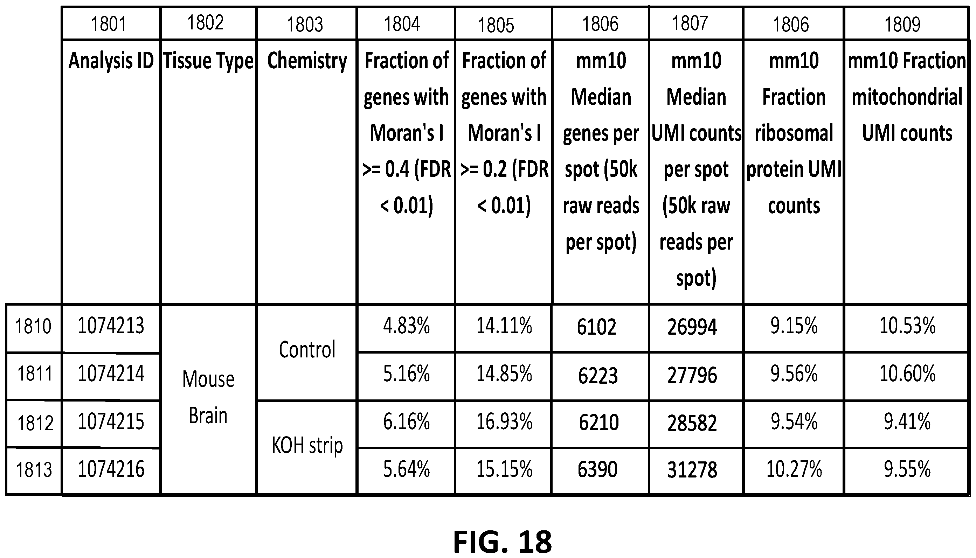

The properties of the two libraries generated using the control workflow **(1810** and **1811)** and the two libraries generated using the test workflow (KOH-Triton) **(1812** and **1813)** is shown in **FIG. 18****.** Library **1810** corresponds to control heat maps **1401** and **1601** and library **1811** corresponds to control heat maps **1402** and **1602** of mouse brain images in **FIGS. 14A** and **16A****.** Library **1812** corresponds to test heat map **1403** and **1603** and library **1813** corresponds to test heat map **1404** and **1604** of mouse brain image in **FIGS. 14B** and **16B****.** The control workflow and the test workflow show comparable sequencing metrics, and libraries **1812** and **1813** show increased mm10 Median genes per spot **1806** and mm10 Median UMI counts per spot **1812.** The data indicate that the methods provided herein provide for an accurate and more efficient option for generating a library.

### EMBODIMENTS

Embodiment 1 is a method comprising the steps of: (a) contacting a biological sample with a substrate comprising a plurality of capture probes, wherein a capture probe of the plurality comprises a capture domain; (b) releasing one or more target analyte(s) from the biological sample, wherein a target analyte of the one or more target analyte(s) that is released from the biological sample is specifically bound by the capture domain of the capture probe; (c) extending the capture probe using the target analyte that is specifically bound by the capture domain of the capture probe as a template, to generate an extended capture probe; (d) exposing the extended capture probe to:(i) a base; and (ii) a detergent, wherein the exposing results in release of the extended capture probe of step (c) from the substrate.

Embodiment 2 is the method of embodiment 1, wherein, in step (a), the capture domain is positioned at a 3' end of the capture probe.

Embodiment 3 is the method of embodiment 1 or 2, wherein step (c) comprises extending a 3' end of the capture probe using the target analyte that is specifically bound by the capture domain of the capture probe as a template, to generate the extended capture probe.

Embodiment 4 is the method of any one of embodiments 1-3, wherein step (c) comprises the use of a reverse transcriptase.

Embodiment 5 is the method of any one of embodiments 1-4, wherein the detergent is a nonionic detergent.

Embodiment 6 is the method of embodiment 5, wherein the non-ionic detergent is Triton-X 100.

Embodiment 7 is the method of any one of embodiments 1-4, wherein the detergent is an anionic detergent.

Embodiment 8 is the method of embodiment 7, wherein the anionic detergent is sodium dodecyl sulfate (SDS).

Embodiment 9 is the method of any one of embodiments 1-8, wherein the detergent is present at a concentration of about 0.1% w/v to about 2.0% w/v.

Embodiment 10 is the method of embodiment 9, wherein the detergent is present at a concentration of about 0.5% w/v to about 1.5% w/v.

Embodiment 11 is the method of any one of embodiments 1-10, wherein the base is potassium hydroxide.

Embodiment 12 is the method of any one of embodiments 1-11, wherein the base is present at a concentration of about 0.01 M to about 0.3 M.

Embodiment 13 is the method of embodiment 12, wherein the base is present at a concentration of about 0.05 M to about 0.15 M.

Embodiment 14 is the method of any one of embodiments 1-13 wherein the exposing is performed at a temperature of about 30 °C to about 80 °C.

Embodiment 15 is the method of embodiment 14, wherein the exposing is performed at a temperature of about 40 °C to about 80 °C.

Embodiment 16 is the method of embodiment 15, wherein the exposing is performed at a temperature of about 60 °C to about 70 °C.

Embodiment 17 is the method of any one of embodiments 1-16, wherein the exposing is performed for about 1 minute to about 2 hours.

Embodiment 18 is the method of embodiment 17, wherein the exposing is performed for about 1 minute to about 1 hour.

Embodiment 19 is the method of embodiment 18, wherein the exposing is performed for about 1 minute to about 15 minutes.

Embodiment 20 is the method of any one of embodiments 1-19, wherein the method further comprises, after step (d): (e) disposing the released extended capture probe into a receptacle and (f) adding a neutralizing agent to the receptacle.

Embodiment 21 is the method of embodiment 20, wherein the neutralizing agent is an acid.

Embodiment 22 is the method of embodiment 20, wherein the neutralizing agent is a buffer.

Embodiment 23 is the method of embodiment 22, wherein the buffer is 2-amino-2-(hydroxymethyl)propane-1,3-diol or 2-[4-(2-hydroxyethyl)piperazin-1-yl]ethanesulfonic acid.

Embodiment 24 is the method of any one of embodiments 1-23, wherein the method further comprises after step (d): (e) determining an amount of the extended capture probe released from the substrate.

Embodiment 25 is the method of embodiment 24, wherein the method further comprises comparing the amount of extended capture probe released from the substrate in step (e) to a reference level.

Embodiment 26 is the method of embodiment 25, wherein the reference level is produced by a control method that comprises performance of steps (a) through (d) but uses one or more of: a different biological sample, a different set of reagents and/or conditions in step (b), a different set of reagents and/or conditions in step (c), and a different set of reagents and/or conditions in step (d).

Embodiment 27 is the method of any one of embodiments 1-26, wherein the target analyte is RNA.

Embodiment 28 is the method of embodiment 27, wherein the RNA is mRNA.

Embodiment 29 is the method of embodiment 27, wherein the capture domain comprises a poly(T) sequence.

Embodiment 30 is the method of any one of embodiments 1-25, wherein the target analyte is DNA.

Embodiment 31 is the method of embodiment 30, wherein the DNA is genomic DNA.

Embodiment 32 is the method of any one of embodiments 1-31, wherein the biological sample is a tissue sample.

Embodiment 33 is the method of embodiment 32, wherein the tissue sample is a fixed tissue sample.

Embodiment 34 is the method of embodiment 33, wherein the fixed tissue sample is a formalin-fixed paraffin-embedded (FFPE) sample.

Embodiment 35 is the method of embodiment 32, wherein the tissue sample is a fresh, frozen tissue sample.

Embodiment 36 is the method of any one of embodiments 1-35, wherein the determining in step (f) comprises: amplifying an extended capture probe to generate an amplification product.

Embodiment 37 is the method of embodiment 36, wherein the determining in step (f) further comprises: generating a library using the amplification product.

Embodiment 38 is a kit comprising a substrate comprising a base, a detergent, and a substrate comprising a plurality of capture probes, wherein a capture probe of the plurality comprises a capture domain.

Embodiment 39 is the kit of embodiment 38, wherein the capture domain is positioned at a 3' end of the capture probe.

Embodiment 40 is the kit of embodiment 38 or 39, further comprising a reverse transcriptase.

Embodiment 41 is the kit of any one of embodiments 38-40, wherein the detergent is a non-ionic detergent.

Embodiment 42 is the kit of embodiment 41, wherein the non-ionic detergent is Triton-X 100.

Embodiment 43 is the kit of any one of embodiments 38-40, wherein the detergent is an anionic detergent.

Embodiment 44 is the kit of embodiment 43, wherein the anionic detergent is sodium dodecyl sulfate (SDS).

Embodiment 45 is the kit of any one of embodiments 38-44, wherein the detergent is present at a concentration of about 0.1% w/v to about 2.0% w/v.

Embodiment 46 is the kit of embodiment 45, wherein the detergent is present at a concentration of about 0.5% w/v to about 1.5% w/v.

Embodiment 47 is the kit of any one of embodiments 38-46, wherein the base is potassium hydroxide.

Embodiment 48 is the kit of any one of embodiments 38-47, wherein the base is present at a concentration of about 0.01 M to about 0.3 M.

Embodiment 49 is the kit of embodiment 48, wherein the base is present at a concentration of about 0.05 M to about 0.15 M.

Embodiment 50 is the kit of any one of embodiments 38-49, wherein the kit further comprises a neutralizing agent.

Embodiment 51 is the kit of embodiment 50, wherein the neutralizing agent is an acid.

Embodiment 52 is the kit of embodiment 50, wherein the neutralizing agent is a buffer.

Embodiment 53 is the kit of embodiment 52, wherein the buffer is 2-amino-2-(hydroxymethyl)propane-1,3-diol or 2-[4-(2-hydroxyethyl)piperazin-1-yl]ethanesulfonic acid.

Embodiment 54 is the kit of any one of embodiments 38-53, wherein the kit further comprises instructions for performing a method of any one of embodiments 1-37.

Embodiment 55 is a method of determining a location of a target analyte in a biological sample, the method comprising: (a) contacting the biological sample with a substrate comprising a plurality of capture probes, wherein a capture probe of the plurality comprises a capture domain and a spatial barcode; (b) releasing one or more target analyte(s) from the biological sample, wherein a target analyte of the one or more target analyte(s) that is released from the biological sample is specifically bound by the capture domain of the capture probe; (c) extending an end of the capture probe using the target analyte that is specifically bound by the capture domain of the capture probe as a template, to generate an extended capture probe; (d) exposing the capture probe to: (i) a base; and (ii) a detergent, wherein the exposing results in release of the extended capture probe of step (c) from the substrate; (e) adding a neutralizing agent; and (1) determining (i) all or a part of a sequence corresponding to the target analyte specifically bound by the capture domain or a complement thereof, and (ii) all or a part of a sequence corresponding to the spatial barcode or complement thereof, and using the determined sequences of (i) and (ii) to determine the location of the target analyte in the biological sample.

Embodiment 56 is the method of embodiment 55, wherein the method further comprises, between steps (d) and (e), disposing the released extended capture probe into a receptacle, and step (e) comprises adding the neutralizing agent to the receptacle.

Embodiment 57 is the method of embodiment 55 or 56, wherein, in step (a), the capture domain is positioned at a 3' end of the capture probe.

Embodiment 58 is the method of embodiment any one of embodiments 55-57, wherein the capture probe further comprises a unique molecular identifier (UMI).

Embodiment 59 is the method of embodiment 58, wherein the UMI is positioned 5' relative to the capture domain.

Embodiment 60 is the method of embodiment any one of embodiments 55-59, wherein step (c) comprises extending a 3' end of the capture probe using the target analyte that is specifically bound by the capture domain of the capture probe as a template, to generate the extended capture probe.

Embodiment 61 is the method of any one of embodiments 55-60, wherein step (c) comprises the use of a reverse transcriptase.

Embodiment 62 is the method of any one of embodiments 55-61, wherein the detergent is a non-ionic detergent.

Embodiment 63 is the method of embodiment 62, wherein the non-ionic detergent is Triton-X 100.

Embodiment 64 is the method of any one of embodiments 55-61, wherein the detergent is an anionic detergent.

Embodiment 65 is the method of embodiment 64, wherein the anionic detergent is sodium dodecyl sulfate (SDS).

Embodiment 66 is the method of any one of embodiments 55-65, wherein the detergent is present at a concentration of about 0.1% w/v to about 2.0% w/v.

Embodiment 67 is the method of embodiment 66, wherein the detergent is present at a concentration of about 0.5% w/v to about 1.5% w/v.

Embodiment 68 is the method of any one of embodiments 55-67, wherein the base is potassium hydroxide.

Embodiment 69 is the method of any one of embodiments 55-68, wherein the base is present at a concentration of about 0.01 M to about 0.3 M.

Embodiment 70 is the method of embodiment 69, wherein the base is present at a concentration of about 0.05 M to about 0.15 M.

Embodiment 71 is the method of any one of embodiments 55-70, wherein the exposing is performed at a temperature of about 30 °C to about 80 °C.

Embodiment 72 is the method of embodiment 71, wherein the exposing is performed at a temperature of about 40 °C to about 80 °C.

Embodiment 73 is the method of embodiment 72, wherein the exposing is performed at a temperature of about 60 °C to about 70 °C.

Embodiment 74 is the method of any one of embodiments 55-73, wherein the exposing is performed for about 1 minute to about 2 hours.

Embodiment 75 is the method of embodiment 74, wherein the exposing is performed for about 1 minute to about 1 hour.

Embodiment 76 is the method of embodiment 75, wherein the exposing is performed for about 1 minute to about 15 minutes.

Embodiment 77 is the method of any one of embodiments 55-76, wherein the neutralizing agent is an acid.

Embodiment 78 is the method of any one of embodiments 55-77, wherein the neutralizing agent is a buffer.

Embodiment 79 is the method of embodiment 78, wherein the buffer is 2-amino-2-(hydroxymethyl)propane-1,3-diol or 2-[4-(2-hydroxyethyl)piperazin-1-yl]ethanesulfonic acid.

Embodiment 80 is the method of any one of embodiments 55-79, wherein the determining in step (f) comprises sequencing (i) all or a part of the sequence corresponding to the target analyte specifically bound by the capture domain or the complement thereof, and (ii) all or a part of the sequence corresponding to the spatial barcode or the complement thereof.

Embodiment 81 is the method of embodiment 80, wherein the sequencing is high throughput sequencing.

Embodiment 82 is the method of any one of embodiments 55-81, wherein the target analyte is a RNA.

Embodiment 83 is the method of embodiment 82, wherein the RNA is mRNA.

Embodiment 84 is the method of embodiment 83, wherein the capture domain comprises a poly(T) sequence.

Embodiment 85 is the method of any one of embodiments 55-81, wherein the target analyte is DNA.

Embodiment 86 is the method of embodiment 85, wherein the DNA is genomic DNA.

Embodiment 87 is the method of any one of embodiments 55-81, wherein the biological sample is a tissue sample.

Embodiment 88 is the method of embodiment 87, wherein the tissue sample is a fixed tissue sample.

Embodiment 89 is the method of embodiment 88, wherein the fixed tissue sample is a formalin-fixed paraffin-embedded (FFPE) sample.

Embodiment 90 is the method of embodiment 87, wherein the tissue sample is a fresh, frozen tissue sample.

Embodiment 91 is the method of any one of embodiments 55-90, wherein the determining in step (f) comprises: amplifying an extended capture probe to generate an amplification product.

Embodiment 92 is the method of embodiment 91, wherein the determining in step (1) further comprises: generating a library using the amplification product.

Embodiment 93 is a method comprising: (a) contacting a biological sample with a plurality of analyte capture agents, wherein an analyte capture agent of the plurality of analyte capture agents comprises an analyte binding moiety, analyte binding moiety barcode, and an analyte capture sequence; (b) contacting the biological sample with a substrate comprising a plurality of capture probes, wherein a capture probe of the plurality comprises a capture domain that binds specifically to the analyte capture sequence; (c) releasing one or more target analyte(s) from the biological sample, wherein a target analyte of the one or more target analyte(s) that is released from the biological sample is specifically bound by the analyte binding moiety of the analyte capture agent and the analyte capture sequence is specifically bound by the capture domain; (d) extending an end of the capture probe using the analyte capture sequence that is specifically bound by the capture domain of the capture probe as a template, to generate an extended capture probe; (e) exposing the capture probe to: (i) a base; and (ii) a detergent, wherein the exposing results in release of the extended capture probe of step (d) from the substrate.

Embodiment 94 is the method of embodiment 93, wherein, in step (b), the capture domain is positioned at a 3' end of the capture probe.

Embodiment 95 is the method of embodiment 93 or 94, wherein step (d) comprises extending a 3' end of the capture probe using the analyte capture sequence that is specifically bound by the capture domain of the capture probe as a template, to generate the extended capture probe.

Embodiment 96 is the method of any one of embodiments 93-95, wherein step (d) comprises the use of a DNA polymerase.

Embodiment 97 is the method of any one of embodiments 93-96, wherein the detergent is a non-ionic detergent.

Embodiment 98 is the method of embodiment 97, wherein the non-ionic detergent is Triton-X 100.

Embodiment 99 is the method of any one of embodiments 93-96, wherein the detergent is an anionic detergent.

Embodiment 100 is the method of embodiment 99, wherein the anionic detergent is sodium dodecyl sulfate (SDS).

Embodiment 101 is the method of any one of embodiments 93-100, wherein the detergent is present at a concentration of about 0.1% w/v to about 2.0% w/v.

Embodiment 102 is the method of embodiment 101, wherein the detergent is present at a concentration of about 0.5% w/v to about 1.5% w/v.

Embodiment 103 is the method of any one of embodiments 93-102, wherein the base is potassium hydroxide.

Embodiment 104 is the method of any one of embodiments 93-103, wherein the base is present at a concentration of about 0.01 M to about 0.3 M.

Embodiment 105 is the method of embodiment 104, wherein the base is present at a concentration of about 0.05 M to about 0.15 M.

Embodiment 106 is the method of any one of embodiments 93-105, wherein the exposing is performed at a temperature of about 30 °C to about 80 °C.

Embodiment 107 is the method of embodiment 106, wherein the exposing is performed at a temperature of about 40 °C to about 80 °C.

Embodiment 108 is the method of embodiment 107, wherein the exposing is performed at a temperature of about 60 °C to about 70 °C.

Embodiment 109 is the method of any one of embodiments 93-108, wherein the exposing is performed for about 1 minute to about 2 hours.

Embodiment 110 is the method of embodiment 109, wherein the exposing is performed for about 1 minute to about 1 hour.

Embodiment 111 is the method of embodiment 110, wherein the exposing is performed for about 1 minute to about 15 minutes.

Embodiment 112 is the method of any one of embodiments 93-111, wherein the method further comprises, after step (d): (e) disposing the released extended capture probe into a receptacle and (f) adding a neutralizing agent to the receptacle.

Embodiment 113 is the method of embodiment 112, wherein the neutralizing agent is an acid.

Embodiment 114 is the method of embodiment 113, wherein the neutralizing agent is a buffer.

Embodiment 115 is the method of embodiment 114, wherein the buffer is 2-amino-2-(hydroxymethyl)propane-1,3-diol or 2-[4-(2-hydroxyethyl)piperazin-1-yl]ethanesulfonic acid.

Embodiment 116 is the method of any one of embodiments 93-115, wherein the method further comprises after step (e): (i) determining an amount of the extended capture probe released from the substrate.

Embodiment 117 is the method of embodiment 116, wherein the method further comprises comparing the amount of extended capture probe released from the substrate in step (i) to a reference level.

Embodiment 118 is the method of embodiment 117, wherein the reference level is produced by a control method that comprises performance of steps (a) through (e) but uses one or more of: a different biological sample, a different set of reagents and/or conditions in step (a), a different set of reagents and/or conditions in step (b), a different set of reagents and/or conditions in step (c), a different set of reagents and/or conditions in step (d), and a different set of reagents and/or conditions in step (e).

Embodiment 119 is the method of any one of embodiments 93-118, wherein the target analyte is a protein.

Embodiment 120 is the method of embodiment 119, wherein the protein is an intracellular protein.

Embodiment 121 is the method of embodiment 119 or 120, wherein the analyte binding moiety comprises an antibody or an antigen-binding fragment thereof.

Embodiment 122 is the method of any one of embodiments 93-121, wherein the biological sample is a tissue sample.

Embodiment 123 is the method of embodiment 122, wherein the tissue sample is a fixed tissue sample.

Embodiment 124 is the method of embodiment 123, wherein the fixed tissue sample is a formalin-fixed paraffin-embedded (FFPE) sample.

Embodiment 125 is the method of embodiment 122, wherein the tissue sample is a fresh, frozen tissue sample.

Embodiment 126 is the method of any one of embodiments 93-125, wherein the determining in step (f) comprises: amplifying an extended capture probe to generate an amplification product.

Embodiment 127 is the method of embodiment 126, wherein the determining in step (f) further comprises: generating a library using the amplification product.

Embodiment 128 is a kit comprising: a plurality of analyte capture agents, wherein an analyte capture agent of the plurality of analyte capture agents comprises an analyte binding moiety, analyte binding moiety barcode, and an analyte capture sequence; a substrate comprising a plurality of capture probes, wherein a capture probe of the plurality comprises a capture domain that binds specifically to the analyte capture sequence; a base; and a detergent.

Embodiment 129 is the kit of embodiment 128, wherein the capture domain is positioned at a 3' end of the capture probe.

Embodiment 130 is the kit of embodiment 128 or 129, wherein the kit further comprises a DNA polymerase.

Embodiment 131 is the kit of any one of embodiments 128-130, wherein the detergent is a non-ionic detergent.

Embodiment 132 is the kit of embodiment 131, wherein the non-ionic detergent is Triton-X 100.

Embodiment 133 is the kit of any one of embodiments 128-130, wherein the detergent is an anionic detergent.

Embodiment 134 is the kit of embodiment 133, wherein the anionic detergent is sodium dodecyl sulfate (SDS).

Embodiment 135 is the kit of any one of embodiments 128-134, wherein the detergent is present at a concentration of about 0.1% w/v to about 2.0% w/v.

Embodiment 136 is the kit of embodiment 135, wherein the detergent is present at a concentration of about 0.5% w/v to about 1.5% w/v.

Embodiment 137 is the kit of any one of embodiments 128-136, wherein the base is potassium hydroxide.

Embodiment 138 is the kit of any one of embodiments 128-137, wherein the base is present at a concentration of about 0.01 M to about 0.3 M.

Embodiment 139 is the kit of embodiment 138, wherein the base is present at a concentration of about 0.05 M to about 0.15 M.

Embodiment 140 is the kit of any one of embodiments 128-139, wherein the kit further comprises a neutralizing agent.

Embodiment 141 is the kit of embodiment 140, wherein the neutralizing agent is an acid.

Embodiment 142 is the kit of embodiment 140, wherein the neutralizing agent is a buffer.

Embodiment 143 is the kit of embodiment 142, wherein the buffer is 2-amino-2-(hydroxymethyl)propane-1,3-diol or 2-[4-(2-hydroxyethyl)piperazin-1-yl]ethanesulfonic acid.

Embodiment 144 is the kit of any one of embodiments 128-143, wherein the analyte binding moiety comprises an antibody or an antigen-binding fragment thereof.

Embodiment 145 is the kit of embodiment 128-144, wherein the kit further comprises instructions for performing a method of any one of embodiments 93-127.

Embodiment 146 is a method of determining a location of a target analyte(s) in a biological sample, the method comprising: (a) contacting a biological sample with a plurality of analyte capture agents, wherein an analyte capture agent of the plurality of analyte capture agents comprises an analyte binding moiety, analyte binding moiety barcode, and an analyte capture sequence; (b) contacting the biological sample with a substrate comprising a plurality of capture probes, wherein a capture probe of the plurality comprises a spatial barcode and a capture domain that binds specifically to the analyte capture sequence; (c) releasing one or more target analyte(s) from the biological sample, wherein a target analyte of the one or more target analyte(s) that is released from the biological sample is specifically bound by the analyte binding moiety of the analyte capture agent and the analyte capture sequence is specifically bound by the capture domain; (d) extending an end of the capture probe using the target analyte that is specifically bound by the analyte capture sequence of the capture probe as a template, to generate an extended capture probe; (e) exposing the capture probe to: (i) a base; and (ii) a detergent, wherein the exposing results in release of the extended capture probe of step (d) from the substrate (1) adding a neutralizing agent; and (g) determining (i) all or a part of a sequence corresponding to the analyte binding moiety barcode or a complement thereof, and (ii) all or a part of a sequence corresponding to the spatial barcode or a complement thereof, and using the determined sequences of (i) and (ii) to determine the location of the target analyte in the biological sample.

Embodiment 147 is the method of embodiment 146, wherein the method further comprises, between steps (e) and (f), disposing the released extended capture probe into a receptacle, and step (f) comprises adding the neutralizing agent to the receptacle.

Embodiment 148 is the method of embodiment 146 or 147, wherein, in step (b), the capture domain is positioned at a 3' end of the capture probe.

Embodiment 149 is the method of any one of embodiments 146-148, wherein the capture probe further comprises a unique molecular identifier (UMI).

Embodiment 150 is the method of embodiment 149, wherein the UMI is positioned 5' relative to the capture domain.

Embodiment 151 is the method of any one of embodiments 146-150, wherein step (d) comprises extending a 3' end of the analyte capture sequence using the target analyte that is specifically bound by the capture domain of the capture probe as a template, to generate the extended capture probe.

Embodiment 152 is the method of any one of embodiments 146-151, wherein step (d) comprises the use of a DNA polymerase

Embodiment 153 is the method of any one of embodiments 146-152, wherein the detergent is a non-ionic detergent.

Embodiment 154 is the method of embodiment 153, wherein the non-ionic detergent is Triton-X 100.

Embodiment 155 is the method of any one of embodiments 146-152, wherein the detergent is an anionic detergent.

Embodiment 156 is the method of embodiment 155, wherein the anionic detergent is sodium dodecyl sulfate (SDS).

Embodiment 157 is the method of any one of embodiments 146-156, wherein the detergent is present at a concentration of about 0.1% w/v to about 2.0% w/v.

Embodiment 158 is the method of embodiment 157, wherein the detergent is present at a concentration of about 0.5% w/v to about 1.5% w/v.

Embodiment 159 is the method of any one of embodiments 146-158, wherein the base is potassium hydroxide.

Embodiment 160 is the method of any one of embodiments 146-159, wherein the base is present at a concentration of about 0.01 M to about 0.3 M.

Embodiment 161 is the method of embodiment 160, wherein the base is present at a concentration of about 0.05 M to about 0.15 M.

Embodiment 162 is the method of any one of embodiments 146-161, wherein the exposing is performed at a temperature of about 30 °C to about 80 °C.

Embodiment 163 is the method of embodiment 162, wherein the exposing is performed at a temperature of about 40 °C to about 80 °C.

Embodiment 164 is the method of embodiment 163, wherein the exposing is performed at a temperature of about 60 °C to about 70 °C.

Embodiment 165 is the method of any one of embodiments 146-164, wherein the exposing is performed for about 1 minute to about 2 hours.

Embodiment 166 is the method of embodiment 165, wherein the exposing is performed for about 1 minute to about 1 hour.

Embodiment 167 is the method of embodiment 166, wherein the exposing is performed for about 1 minute to about 15 minutes.

Embodiment 168 is the method of embodiment 146, wherein the neutralizing agent is an acid.

Embodiment 169 is the method of embodiment 146, wherein the neutralizing agent is a buffer.

Embodiment 170 is the method of embodiment 146, wherein the buffer is 2-amino-2-(hydroxymethyl)propane-1,3-diol or 2-[4-(2-hydroxyethyl)piperazin-1-yl]ethanesulfonic acid.

Embodiment 171 is the method of any one of embodiments 146-170, wherein the determining in step (g) comprises sequencing all or a part of the sequence corresponding to the analyte binding moiety barcode or the complement thereof, and (ii) all or a part of the sequence corresponding to the spatial barcode or the complement thereof.

Embodiment 172 is the method of embodiment 171, wherein the sequencing is high throughput sequencing.

Embodiment 173 is the method of any one of embodiments 146-172, wherein the target analyte is a protein.

Embodiment 174 is the method of embodiment 173, wherein the protein is an intracellular protein.

Embodiment 175 is the method of embodiment 173 or 174, wherein the analyte binding moiety comprises an antibody or an antigen-binding fragment thereof.

Embodiment 176 is the method of any one of embodiments 146-175, wherein the biological sample is a tissue sample.

Embodiment 177 is the method of embodiment 176, wherein the tissue sample is a fixed tissue sample.

Embodiment 178 is the method of embodiment 177, wherein the fixed tissue sample is a formalin-fixed paraffin-embedded (FFPE) sample.

Embodiment 179 is the method of embodiment 176, wherein the tissue sample is a fresh, frozen tissue sample.

Embodiment 180 is the method of any one of embodiments 146-179, wherein the determining in step (f) comprises: amplifying an extended capture probe to generate an amplification product.

Embodiment 181 is the method of embodiment 180, wherein the determining in step (f) further comprises: generating a library using the amplification product.

### NUMBERED EMBODIMENTS

1. A method of determining a location of a target analyte in a biological sample, the method comprising:
   (a) contacting the biological sample with an array comprising a plurality of capture probes, wherein a capture probe of the plurality comprises (i) a capture domain and (ii) a spatial barcode;
   (b) releasing one or more target analyte(s) from the biological sample, wherein a target analyte of the one or more target analyte(s) that is released from the biological sample is hybridized by the capture domain of the capture probe;
   (c) extending an end of the capture probe using the target analyte hybridized to the capture domain of the capture probe as a template, to generate an extended capture probe;
   (d) exposing the capture probe to:
      (i) a base; and
      (ii) a detergent, wherein the exposing results in release of the extended capture probe of step (c) from the array;
   (e) adding a neutralizing agent; and
   (f) determining
      (i) all or a part of a sequence corresponding to the target analyte hybridized to the capture domain or a complement thereof, and
      (ii) all of a sequence corresponding to the spatial barcode or complement thereof, and using the determined sequences of (i) and (ii) to determine the location of the target analyte in the biological sample.
2. The method of embodiment 1, wherein the method further comprises, between steps (d) and (e), disposing the released extended capture probe into a receptacle, and step (e) comprises adding the neutralizing agent to the receptacle.
3. The method of embodiment 1 or 2, wherein, in step (a), the capture domain is positioned at a 3' end of the capture probe.
4. The method of any one of embodiments 1-3, wherein the capture probe further comprises a unique molecular identifier (UMI) and the UMI is positioned 5' relative to the capture domain.
5. The method of any one of embodiments 1-4, wherein step (c) comprises extending a 3' end of the capture probe using the target analyte hybridized to the capture domain of the capture probe as a template, to generate the extended capture probe.
6. The method of any one of embodiments 1-5, 6. wherein step (c) comprises the use of a reverse transcriptase.
7. The method of any one of embodiments 1-6, wherein the detergent is a non-ionic detergent.
8. The method of embodiment 7, wherein the non-ionic detergent is Triton-X 100.
9. The method of any one of embodiments 1-6, wherein the detergent is an anionic detergent.
10. The method of embodiment 9, wherein the anionic detergent is sodium dodecyl sulfate (SDS).
11. The method of any one of embodiments 1-10, wherein the detergent is present at a concentration of about 0.1% w/v to about 2.0% w/v.
12. The method of any one of embodiments 1-11, wherein the base is potassium hydroxide.
13. The method of any one of embodiments 1-12, wherein the base is present at a concentration of about 0.01 M to about 0.3 M.
14. The method of any one of embodiments 1-13, wherein the exposing is performed at a temperature of about 30 °C to about 80 °C.
15. The method of any one of embodiments 1-14, wherein the exposing is performed for about 1 minute to about 2 hours.
16. The method of any one of embodiments 1-15, wherein the neutralizing agent is an acid.
17. The method of any one of embodiments 1-16, wherein the neutralizing agent is a buffer.
18. The method of embodiment 17, wherein the buffer is 2-amino-2-(hydroxymethyl) propane-1,3-diol or 2-[4-(2-hydroxyethyl)piperazin-1-yl]ethanesulfonic acid.
19. The method of any one of embodiments 1-18, wherein the determining in step (f) comprises sequencing (i) all or a part of the sequence corresponding to the target analyte specifically hybridized to the capture domain or the complement thereof, and (ii) all or a part of the sequence corresponding to the spatial barcode or the complement thereof.
20. The method of embodiment 19, wherein the sequencing is high throughput sequencing.
21. The method of any one of embodiments 1-20, wherein the target analyte is an RNA or an mRNA.
22. The method of embodiment 21, wherein the capture domain comprises a poly(T) sequence.
23. The method of any one of embodiments 1-20, wherein the target analyte is DNA or genomic DNA.
24. The method of any one of embodiments 1-23, wherein the biological sample is a tissue sample, a tissue section or a fixed tissue section, and optionally wherein the fixed tissue section is a formalin-fixed paraffin-embedded tissue section or the tissue section is a fresh, frozen tissue section.
25. The method of any one of embodiments 1-24, wherein the determining in step (1) comprises: amplifying an extended capture probe to generate an amplification product.
26. The method of embodiment 25, wherein the determining in step (f) further comprises: generating a library using the amplification product.
27. A kit comprising a substrate comprising a base, a detergent, and a substrate comprising a plurality of capture probes, wherein a capture probe of the plurality comprises a capture domain.
28. The kit of embodiment 27, wherein the capture 28. domain is positioned at a 3' end of the capture probe.
29. The kit of embodiment 27 or 28, further comprising a reverse transcriptase.
30. The kit of any one of embodiments 27-29, wherein the detergent is a non-ionic detergent.
31. The kit of embodiment 30, wherein the non-ionic detergent is Triton-X 100.
32. The kit of any one of embodiments 27-29, wherein the detergent is an anionic detergent.
33. The kit of embodiment 32, wherein the anionic detergent is sodium dodecyl sulfate (SDS).
34. The kit of any one of embodiments 27-33, wherein the detergent is present at a concentration of about 0.1% w/v to about 2.0% w/v.
35. The kit of embodiment 34, wherein the detergent is present at a concentration of about 0.5% w/v to about 1.5% w/v.
36. The kit of any one of embodiments 27-35, wherein the base is potassium hydroxide.
37. The kit of any one of embodiments 27-36, wherein the base is present at a concentration of about 0.01 M to about 0.3 M.
38. The kit of embodiment 37, wherein the base is present at a concentration of about 0.05 M to about 0.15 M.
39. The kit of any one of embodiments 27-38, wherein the kit further comprises a neutralizing agent.
40. The kit of embodiment 39, wherein the neutralizing agent is an acid.
41. The kit of embodiment 39, wherein the neutralizing agent is a buffer.
42. The kit of embodiment 41, wherein the buffer is 2-ammo-2-(hydroxymethyl) propane-1,3-diol or 2-[4-(2-hydroxyethyl)piperazin-1-yl]ethanesulfonic acid.
43. The kit of any one of embodiments 27-42, wherein the kit further comprises instructions for performing a method of any one of embodiments 1-26.

## Claims

1. A method for releasing an extended capture probe from a spatially barcoded array, wherein the method comprises:
(a) contacting a biological sample with:
(i) an analyte capture agent comprising (1) an analyte binding moiety that binds to a target analyte, (2) an analyte binding moiety barcode, and (3) an analyte capture sequence, and
(ii) an array comprising a plurality of capture probes, wherein a capture probe of the plurality of capture probes comprises: (1) a capture domain that binds to the analyte capture sequence and (2) a spatial barcode;
(b) extending an end of the capture probe using the analyte binding moiety barcode as a template, to generate an extended capture probe; and
(c) exposing the extended capture probe to:
(i) a base; and
(ii) a detergent,
wherein the exposing results in release of the extended capture probe from the array.

2. The method of claim 1, wherein the capture probe further comprises a unique molecular identifier (UMI) and the UMI is positioned 5' relative to the capture domain.

3. The method of claim 1 or 2, wherein the extending the end of the capture probe in (b) comprises use of a reverse transcriptase.

4. The method of any one of claims 1-3, wherein the detergent is a non-ionic detergent or an anionic detergent, optionally wherein the non-ionic detergent is Triton-X 100 or the anionic detergent is sodium dodecyl sulfate (SDS).

5. The method of any one of claims 1-4, wherein the detergent is present at a concentration of about 0.1% w/v to about 2.0% w/v.

6. The method of any one of claims 1-5, wherein the base is potassium hydroxide or sodium hydroxide.

7. The method of any one of claims 1-6, wherein the base is present at a concentration of about 0.01 M to about 0.3 M.

8. The method of any one of claims 1-7, wherein the exposing is performed for about 1 minute to about 2 hours.

9. The method of any one of claims 1-8, further comprising adding a neutralizing agent to the released extended capture probe, optionally, wherein the neutralizing agent is an acid or a buffer, optionally, wherein the buffer is 2-amino-2-(hydroxymethyl) propane-1,3-diol or 2-[4-(2-hydroxyethyl)piperazin-1-yl]ethanesulfonic acid.

10. The method of claim 9, further comprising disposing the released extended capture probe into a receptacle, and adding the neutralizing agent into the receptacle.

11. The method of any one of claims 1-10, further comprising determining:
(i) a sequence corresponding to the analyte binding moiety barcode or a complement thereof, and
(ii) a sequence corresponding to the spatial barcode or a complement thereof, and using the determined sequences of (i) and (ii) to determine a location of the target analyte in the biological sample.

12. The method of claim 11, wherein the determining comprises sequencing (i) the sequence corresponding to the analyte binding moiety barcode or the complement thereof, and (ii) the sequence corresponding to the spatial barcode or the complement thereof, optionally, wherein the sequencing is high throughput sequencing.

13. The method of any one of claims 1-12, wherein the target analyte is a protein and the analyte binding moiety is an antibody or antigen-binding fragment thereof, optionally, wherein the protein is an extracellular protein, a cell surface protein, or an intracellular protein.

14. The method of any one of claims 1-13, wherein the biological sample is a fresh frozen tissue section, or a fixed tissue section, and optionally, wherein the fixed tissue section is a formalin-fixed paraffin-embedded tissue section.

15. The method of claim 11, wherein the determining comprises: amplifying the extended capture probe to generate an amplification product, and optionally, further comprises generating a library using the amplification product.
